(19) Europäisches Patentamt
European Patent Office
Office européen des brevets

(11) **EP 4 772 103 A1**

(12) **EUROPEAN PATENT APPLICATION**
published in accordance with Art. 153(4) EPC

(43) Date of publication:
**08.07.2026 Bulletin 2026/28**

(21) Application number: **24859146.3**

(22) Date of filing: **01.07.2024**

(51) International Patent Classification (IPC):
**A61B 1/00** $^{(2006.01)}$      **A61B 1/045** $^{(2006.01)}$

(52) Cooperative Patent Classification (CPC):
**A61B 1/00; A61B 1/045**

(86) International application number:
**PCT/JP2024/023815**

(87) International publication number:
**WO 2025/047101 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.08.2023 JP 2023138745**

(71) Applicant: **Sony Olympus Medical Solutions Inc.
Hachioji-shi, Tokyo 192-0904 (JP)**

(72) Inventors:
- **DEGUCHI, Tatsuya
  Hachioji-shi, Tokyo 192-0904 (JP)**
- **MITSUI, Satoshi
  Hachioji-shi, Tokyo 192-0904 (JP)**

(74) Representative: **D Young & Co LLP
3 Noble Street
London EC2V 7BQ (GB)**

(54) **MEDICAL IMAGE PROCESSING DEVICE AND MEDICAL OBSERVATION SYSTEM**

(57)    A medical image processing device 9 includes an image processing unit 92 that processes a pixel signal acquired from a pixel of an image sensor 513. The pixel signal includes a first pixel signal and a second pixel signal. The first pixel signal includes a component of unnecessary light and a component of observation target fluorescence. The unnecessary light includes autofluorescence which is generated from a member forming an observation optical path by light emitted from a light source device when the light propagates through the observation optical path. The component of observation target fluorescence is emitted from a substance contained in an observation target excited by the light. The second pixel signal includes at least the component of the unnecessary light. The image processing unit 92 includes a signal correction unit 923 that generates a corrected pixel signal based on a correction coefficient set based on a spectral characteristic of the observation target fluorescence and based on the first and second pixel signals.

FIG.2

## Description

Field

The present disclosure relates to a medical image processing device and a medical observation system.

Background

Conventionally, there has been known a medical observation system (refer to Patent Literature 1, for example) that irradiates an observation target (subject such as a person) with first light including visible light such as excitation light being narrowband light or white light being broadband light emitted from a light source device, and observes, by the application of the excitation light, fluorescence emitted from a substance contained in the observation target (hereinafter, denoted as observation target fluorescence).

With such fluorescence observation, tissue conditions that are difficult to recognize with white light can be grasped through observation target fluorescence. Accordingly, fluorescence observation can be utilized for various purposes and applications such as identification of a lesion.

Citation List

Patent Literature

Patent Literature 1: JP 2021-132695 A

Summary

Technical Problem

Meanwhile, the medical observation system has an observation optical path including: a first optical path used for propagation of first light from the light source device to the observation target; and a second optical path used for propagation of second light being return light (including observation target fluorescence) of the first light, returned from the observation target irradiated with the first light. When the first and second light propagate through the observation optical path, application of the first and second light to a member constituting the observation optical path may cause autofluorescence generated from the member. Such autofluorescence may have a wavelength band including a wavelength band of observation target fluorescence (which is a target of fluorescence observation), and can be noise in performing the fluorescence observation.

This leads to a demand for a technique capable of satisfactorily performing fluorescence observation.

The present disclosure has been made in view of the above, and aims to provide a medical image processing device and a medical observation system capable of satisfactorily performing fluorescence observation. Solution to Problem

To solve the above-described problem and achieve the object, a medical image processing device according to the present disclosure includes an image processing unit configured to process a pixel signal acquired from a pixel of an image sensor, wherein the pixel signal includes: a first pixel signal including a component of unnecessary light and a component of observation target fluorescence, the unnecessary light including autofluorescence which is generated from a member forming an observation optical path by light emitted from a light source device when the light propagates through the observation optical path, the component of observation target fluorescence having been emitted from a substance contained in an observation target excited by the light; and a second pixel signal including at least the component of the unnecessary light, and the image processing unit includes a signal correction unit configured to generate a corrected pixel signal based on a correction coefficient, the first pixel signal and the second pixel signal, the correction coefficient having been set based on a spectral characteristic of the observation target fluorescence.

A medical observation system according to the present disclosure includes: a light source device including a first light source configured to emit first light including narrowband light that excites a substance contained in an observation target; an imaging device including an image sensor configured to capture second light that is return light of the first light from the observation target; and a medical image processing device including an image processing unit configured to process a pixel signal corresponding to a pixel of the image sensor, wherein the pixel signal includes: a first pixel signal including a component of unnecessary light and a component of observation target fluorescence, the unnecessary light including autofluorescence which is generated from a member forming an observation optical path by the first light emitted from the light source device when the first light propagates through the observation optical path, the component of observation target fluorescence having been emitted from a substance contained in the observation target excited by the first light; and a second pixel signal including at least the component of the unnecessary light, and the image processing unit includes a signal correction unit configured to generate a corrected pixel signal based on a correction coefficient, the first pixel signal, and the second pixel signal, the correction coefficient having been set based on a spectral characteristic of the observation target fluorescence. Advantageous Effects of Invention

With the medical image processing device and the medical observation system according to the present disclosure, fluorescence observation can be performed satisfactorily.

Brief Description of Drawings

FIG. 1 is a diagram illustrating a configuration of a medical observation system according to an embodiment.
FIG. 2 is a block diagram illustrating a configuration of a camera head and a control device.
FIG. 3 is a diagram illustrating a configuration of a color filter.
FIG. 4 is a diagram illustrating a configuration of a color filter.
FIG. 5 is a diagram illustrating a first modification of the embodiment.
FIG. 6 is a diagram illustrating the first modification of the embodiment.
FIG. 7 is a diagram illustrating the first modification of the embodiment.
FIG. 8 is a diagram illustrating the first modification of the embodiment.
FIG. 9 is a diagram illustrating a second modification of the embodiment.
FIG. 10 is a diagram illustrating the second modification of the embodiment.
FIG. 11 is a diagram illustrating a third modification of the embodiment.
FIG. 12 is a diagram illustrating a fourth modification of the embodiment.
FIG. 13 is a diagram illustrating the fourth modification of the embodiment.
FIG. 14 is a diagram illustrating the fourth modification of the embodiment.
FIG. 15 is a diagram illustrating a fifth modification of the embodiment.
FIG. 16 is a diagram illustrating the fifth modification of the embodiment.
FIG. 17 is a diagram illustrating a sixth modification of the embodiment.
FIG. 18 is a diagram illustrating the sixth modification of the embodiment.
FIG. 19 is a diagram illustrating a seventh modification of the embodiment.
FIG. 20 is a diagram illustrating an eighth modification of the embodiment.
FIG. 21 is a diagram illustrating a ninth modification of the embodiment.
FIG. 22 is a diagram illustrating the ninth modification of the embodiment.

Description of Embodiments

Hereinafter, modes for carrying out the present disclosure (hereinafter referred to as embodiments) will be described with reference to the drawings. Note that the present disclosure is not limited to the embodiments described below. In the drawings, same reference signs are attached to the same portions.

[Configuration of medical observation system]

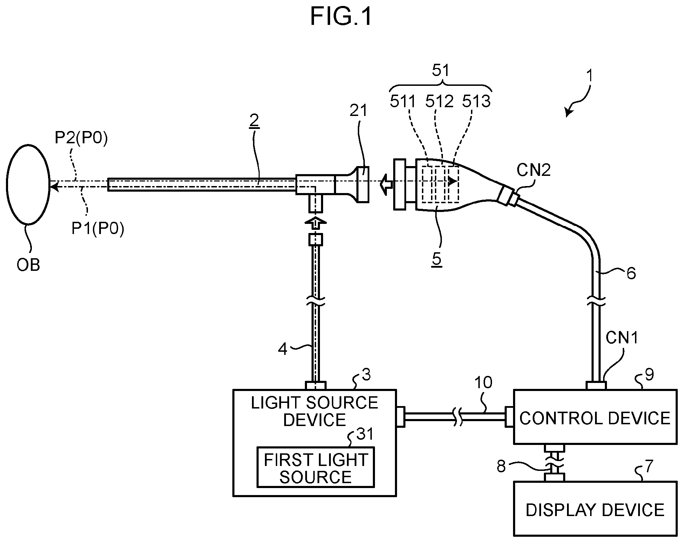

FIG. 1 is a diagram illustrating a configuration of a medical observation system 1 according to the embodiment.
In the present embodiment, the medical observation system 1 is a medical endoscope system that observes an observation target OB within a living body using an endoscope. As illustrated in FIG. 1, the medical observation system 1 includes an insertion unit 2, a light source device 3, a light guide 4, a camera head 5, a first transmission cable 6, a display device 7, a second transmission cable 8, a control device 9, and a third transmission cable 10.
In the present embodiment, the insertion unit 2 is provided as a rigid endoscope. That is, the insertion unit 2 has an elongated shape that is entirely rigid, or partially rigid with a partially flexible portion, so as to be inserted into an observation target OB The insertion unit 2 includes an optical system having one or more lenses and configured to condense second light (subject image) described below from the observation target OB.
The light source device 3 is connected with one end of the light guide 4. The light source device 3 includes a first light source 31 (FIG. 1) that supplies the first light to the one end of the light guide 4 under the control of the control device 9. The present embodiment adopts, as the first light, excitation light (narrowband light) that excites a substance contained in the observation target OB. The first light source 31 may be provided as a light emitting diode (LED) or a semiconductor laser. The number of first light sources 31 that emit the first light may be one or more. Furthermore, the excitation light may be visible light or invisible light. The light source device 3 may include a second light source that emits broadband light such as white light in addition to the first light source 31.

Here, examples of the substance contained in the observation target OB to be excited by the excitation light include an agent or a fluorescent dye applied to the observation target OB or a fluorescent substance derived from the observation target OB, being a substance constituting the observation target OB.

Examples of the above-described agent to be applied to the observation target OB include "5-ALA (PP-IX)", "ADS780WS", "ADS830WS", "aggregation-induced emission dots allophycocyanin (APC)", "boron-dipyrromethane (BODIPY)", "CLR 1502", "Flavins", "fluorescamine", "fluorescein", "fluoro-gold", "green fluorescence protein", "indocyanine green (ICG)", "IRDye 78", "IR-PEG nanoparticles", "Isothiocyanate", "rose Bengal", "SGM-101", and "trypan blue".

In addition, examples of the above-described fluorescent dyes to be applied to the observation target OB include "coumarine", "Cy3", "DyLight547", "GE3126", "metal nanoclusters", "oxacarbocyanine", "Rhodamine", "Riboflavin", "fluorescein", "AlexaFluor660", "AlexaFluor680", "AlexaFluor700", "Cy5", "Cy5.5", "Dy677", "Dy682", "Dy752", "DyLight647", "HiLyte Fluor 647", "HiLyte Fluor 680", "IRDye 700DX", "methylene blue", "Porphyrins", "Porphysomes", "VivoTag-680", "VivoTag-S680", "AlexaFluor750", "AlexaFluor790", "carbocyanine", "conjugated copolymers", "CW800-CA", "Cy7", "Cy7.5", "cyanine dyes", "Dy780", "HiLyte Fluor 750", "Indocarbocyanine", "IR-786", "IRDye 800CW", "IRDye 800RS", "IRDye 800BK", "Nervelight", "OTL-38 (Pafolacianine)", "Polymethine", "VivoTag-S750", "Xanthene", and "LUM-015".

Furthermore, examples of the fluorescent substance derived from the observation target OB constituting the observation target OB itself include "collagen", "elastin", and "NADH".

In the present embodiment, the light source device 3 is separated from the control device 9. However, the configuration is not limited to this, and it is allowable to adopt a configuration in which the light source device 3 is provided inside the same housing as that for the control device 9.

The light guide 4 has its one end detachably connected to the light source device 3. The light guide 4 has its other end detachably connected to the insertion unit 2. The light guide 4 transmits the first light supplied from the light source device 3 (first light source 31) from one end to the other end and supplies the light to the insertion unit 2. The first light supplied to the insertion unit 2 is emitted from a distal end of the insertion unit 2 and applied to the observation target OB. After being applied to the observation target OB, the first light is returned from the observation target OB, and this return light of the first light, defined as the second light (subject image), is condensed by the optical system in the insertion unit 2. The second light includes: the first light reflected by the observation target OB; and fluorescence (hereinafter, denoted as observation target fluorescence) emitted from a substance contained in the observation target OB by excitation of the substance due to the application of the first light (excitation light) to the observation target OB.

The camera head 5 corresponds to an imaging device according to the present disclosure. The camera head 5 is detachably connected to an eyepiece 21 of the insertion unit 2. Under the control of the control device 9, the camera head 5 captures the second light condensed by the insertion unit 2 and generates a pixel signal. Hereinafter, for convenience of description, the pixel signal will be denoted as a captured image.

The detailed configuration of the camera head 5 will be described in "Configuration of camera head" described below.

The first transmission cable 6 has its one end detachably connected to the control device 9 via a connector CN1 (FIG. 1). The first transmission cable 6 has its other end detachably connected to the camera head 5 via a connector CN2 (FIG. 1). The first transmission cable 6 transmits the captured image or the like output from the camera head 5 to the control device 9, and transmits a control signal, a synchronization signal, a clock, power, or the like output from the control device 9 to the camera head 5 individually.

The captured image and the like transmitted from the camera head 5 to the control device 9 via the first transmission cable 6 may be transmitted as an optical signal or may be transmitted as an electrical signal. The similar applies to transmission of the control signal, the synchronization signal, and the clock from the control device 9 to the camera head 5 via the first transmission cable 6.

The display device 7 is implemented by a display using liquid crystal, organic Electro Luminescence (EL), or the like, and displays an image based on a video signal from the control device 9 under the control of the control device 9. Note that the display includes a wearable display.

The second transmission cable 8 has its one end detachably connected to the display device 7. The second transmission cable 8 has its other end detachably connected to the control device 9. The second transmission cable 8 transmits the video signal processed by the control device 9 to the display device 7.

The control device 9 corresponds to the medical image processing device according to the present disclosure. The control device 9 is implemented by a central processing unit (CPU), a graphics processing unit (GPU), a field-programmable gate array (FPGA), or the like, and comprehensively controls operation of the light source device 3, the camera head 5, and the display device 7.

A detailed configuration of the control device 9 will be described in "Configuration of control device" described below.

The third transmission cable 10 has its one end detachably connected to the light source device 3. The third transmission cable 10 has its other end detachably connected to the control device 9. The third transmission cable 10 transmits the control signal from the control device 9 to the light source device 3.

[Configuration of camera head]

Next, a configuration of the camera head 5 will be described.

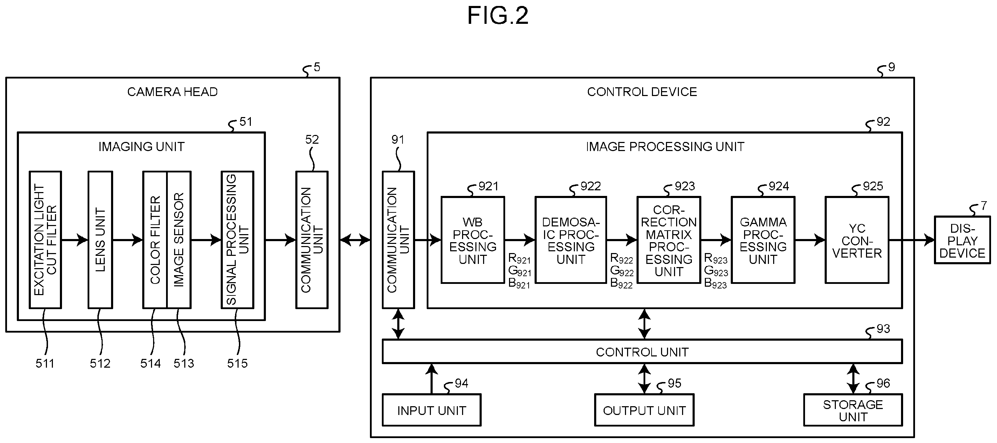

FIG. 2 is a block diagram illustrating a configuration of the camera head 5 and the control device 9.

As illustrated in FIG. 2, the camera head 5 includes an imaging unit 51, and a communication unit 52.

The imaging unit 51 captures the observation target OB under the control of the control device 9. As illustrated in FIG. 2, the imaging unit 51 includes an excitation light cut filter 511, a lens unit 512, an image sensor 513, a color filter 514, and a signal processing unit 515.

The excitation light cut filter 511 is a filter that partially, substantially, or completely suppresses the excitation light contained in the second light (subject image) condensed by the insertion unit 2.

The excitation light cut filter 511 may be disposed in the insertion unit 2 (for example, the eyepiece 21), not limited to the camera head 5.

The lens unit 512 focuses the second light (subject image), which has been condensed by the insertion unit 2 and passing through the excitation light cut filter 511, onto a light receiving surface of the image sensor 513.

In the present embodiment, only one image sensor 513 is provided. The image sensor 513 is provided as a Charge Coupled Device (CCD), Complementary Metal Oxide Semiconductor (CMOS), etc. that receives the second light (subject image), which has passed through the excitation light cut filter 511 and has been focused by the lens unit 512, and converts the received light into an electrical signal. The image sensor 513 captures the second light (subject image) under the control of the control device 9. With this procedure, a pixel signal (captured image) is generated.

The color filter 514 corresponds to a spectral dispersion member according to the present disclosure. The color filter 514 is provided on a light receiving surface of the image sensor 513.

The detailed configuration of the color filter 514 will be described in "Configuration of color filter" described below.

Under the control of the control device 9, the signal processing unit 515 performs signal processing on the captured image (analog signal) generated by the image sensor 513 and outputs the captured image (digital signal).

For example, the signal processing unit 515 performs signal processing such as processing of removing reset noise, processing of multiplying an analog gain to amplify the analog signal, A/D conversion, etc. on the captured image (analog signal) generated on the image sensor 513.

The communication unit 52 functions as a transmitter that sequentially transmits the captured image output from the imaging unit 51 to the control device 9 via the first transmission cable 6. For example, the communication unit 52 includes a high-speed serial interface that performs captured image communication with the control device 9 via the first transmission cable 6 at a transmission rate of 1 Gbps or more. Incidentally, the communication unit 52 may perform communication at a transmission rate lower than 1 Gbps, for example, in a case where the image sensor 513 is an image sensor with HD resolution or lower.

[Configuration of color filter]

Next, a configuration of the color filter 514 will be described.

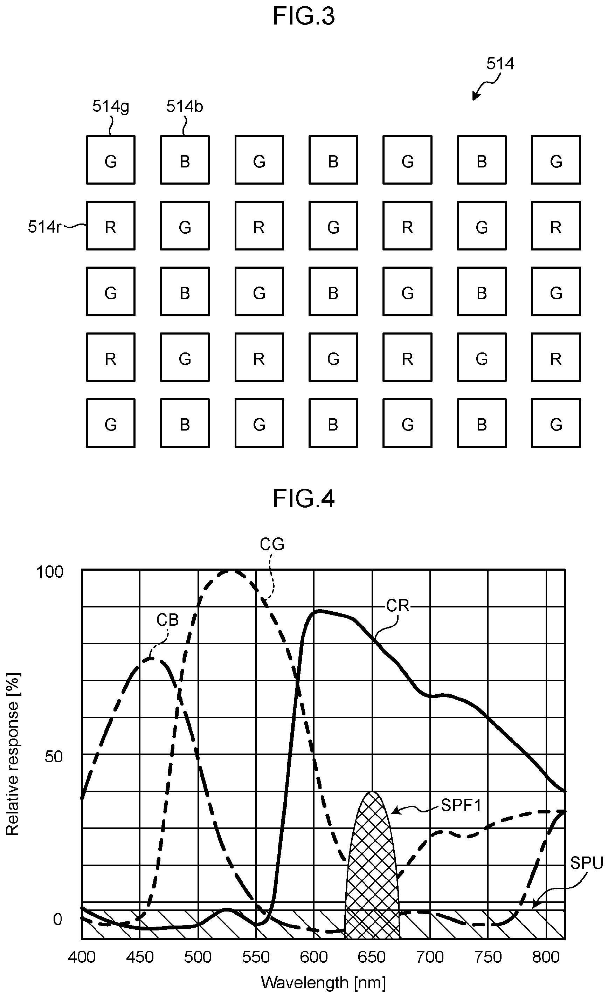

FIGS. 3 and 4 are diagrams illustrating a configuration of the color filter 514. Specifically, FIG. 3 is a diagram illustrating the color filter 514. FIG. 4 is a diagram illustrating spectral characteristics of the color filter 514. The spectral characteristic of the filter such as the color filter 514 is also referred to as a transmission spectral characteristic. In FIG. 4, the horizontal axis represents Wavelength[nm], and the vertical axis represents Relative response [%].

The color filter 514 is a color filter in which three filter groups, which are grouped in accordance with a wavelength band of light to be transmitted (red, green, and blue), are arrayed in a specific format (for example, the Bayer array).

Specifically, as illustrated in FIG. 3, the color filter 514 includes: an R filter group 514r that mainly transmits light in a red wavelength band; a B filter group 514b that mainly transmits light in a blue wavelength band; a first G filter group (arrayed in the same column as the R filter group 514r) that mainly transmits light in a green wavelength band; and a second G filter group (arrayed in the same column as the B filter group 514b) that mainly transmits light in a green wavelength band. In FIG. 3, the first and second G filter groups are collectively denoted as a G filter group 514g. In FIG. 3, the letter "R" is attached to the R filter group 514r, the letter "G" is attached to the G filter group 514g, and the letter "B" is attached to the B filter group 514b. In FIG. 4, the spectral characteristic of the R filter group 514r is indicated by a curve CR, the spectral characteristic of the G filter group 514g is indicated by a curve CG, and the spectral characteristic of the B filter group 514b is indicated by a curve CB. As can be seen from the curves CR, CG, and CB, the spectral characteristics of the filter groups 514r, 514g, and 514b are spectral characteristics overlapping each other in some wavelength bands although they have mutually different transmission peak wavelengths.

In FIG. 4, a reference sign "SPF1" denotes a spectrum of the observation target fluorescence. In FIG. 4, a reference sign "SPU" denotes a wavelength range in which unnecessary light described below can be generated.

The first light emitted from the light source device 3 is applied to the observation target OB via a first optical path P1 (FIG. 1). The first optical path P1 is an optical path that follows the route from the light source device 3, through the light guide 4, the insertion unit 2, to the observation target OB. When the observation target OB is irradiated with the first light, a substance contained in the observation target OB is excited, and observation target fluorescence is emitted from the substance. The observation target fluorescence is observation target light in fluorescence observation. The second light, which is the return light of the first light including the observation target light, is propagated to the image sensor 513 via a second optical path P2 (FIG. 1). The second optical path P2 is an optical path that follows the route from the observation target OB through the insertion unit 2 to the image sensor 513. The excitation light contained in the second light is partially, substantially, or completely suppressed by the excitation light cut filter 511 when propagating through the second optical path P2.

Here, when the first light propagates through the first optical path P1 and when the second light propagates through the second optical path P2, the member forming an observation optical path P0 (FIG. 1) of the first and second light P1 and P2 (hereinafter, denoted as unnecessary light generating member) is irradiated with the first light and the second light, whereby autofluorescence (hereinafter, denoted as unnecessary light) might be generated from the unnecessary light generating member. Examples of the unnecessary light generating member include components contained in multicomponent glass such as an optical fiber and a lens, a material component contained in a color filter, an adhesive bonding a lens to another lens or bonding other optical members to each other, and oil attached to an optical member such as a lens. As illustrated in a wavelength range SPU in which unnecessary light can be generated in FIG. 4, such unnecessary light has a wavelength band including a wavelength band of observation target fluorescence, being observation target light in fluorescence observation, and can be noise in performing the fluorescence observation.

In a case where the observation target fluorescence from the observation target OB is weak in particular, it is necessary to adjust a signal value based on the observation target fluorescence captured by the image sensor 513 in order to separate the observation target fluorescence from the above-described unnecessary light to perform satisfactory fluorescence observation. However, it is difficult to adjust the signal value based on the observation target fluorescence because of the influence of the following (1) to (4).

(1) Agents

Typically, the light amount of observation target fluorescence emitted from an agent varies depending on the type and dosage of the agent.

The type of agent to be administered to the observation target OB is selected in accordance with the observation target OB (such as cancer, blood, and lymph). Furthermore, in order to capture the observation target fluorescence emitted from the agent in the medical observation system 1, the agent to be selected is an agent capable of separating the wavelength of the excitation light for exciting the agent and the wavelength of the observation target fluorescence emitted from the agent. The agents selected in this manner have different light amounts of the observation target fluorescence to be emitted.

In addition, although the light amount of the observation target fluorescence can be adjusted by the dosage of the agent, it is difficult to increase the dosage more than necessary in order to achieve a minimally invasive treatment.

That is, it is difficult to adjust the light amount of the observation target fluorescence by selecting the type of agent and adjusting the dosage. As a result, it is difficult to adjust the signal value based on the observation target fluorescence captured by the image sensor 513 by selecting the type of agent and adjusting the dosage.

(2) Observation target

The light amount of the observation target fluorescence emitted from the observation target OB varies depending on the site/state of the observation target OB.

Specifically, in the case of the observation target OB being the site and having the state in which the agent easily stagnates, the light amount of the observation target fluorescence emitted from the observation target OB increases. In contrast, in the case of the observation target OB being the site and having the state in which the agent easily flows and hardly stagnates, the light amount of the observation target fluorescence emitted from the observation target OB decreases, together with a decrease in the afterglow time. In addition, in a case where the observation target OB is a tumor, the light amount of the observation target fluorescence received by the image sensor 513 varies depending on the spread, size, and depth of the tumor.

That is, it is difficult to adjust the light amount of the observation target fluorescence depending on the type and state of the observation target OB. As a result, it is difficult to adjust the signal value based on the observation target fluorescence imaged by the image sensor 513 depending on the type and state of the observation target OB.

(3) Light source device

The light amount of the observation target fluorescence varies depending on the light amount of the excitation light emitted from the light source device 3.

In order to increase the light amount of the excitation light, it may be necessary to adjust the power supplied to the light source device 3. However, the power that can be supplied to the light source device 3 is limited with the upper limit value of the power for operating the entire medical observation system 1. In addition, the light amount of the excitation light is required to be adjusted in consideration of conditions such as: heat generation (for example, heat generation between the location such as the light guide 4 and the insertion unit 2) in a member constituting an optical path of the excitation light; conformity to the laser class; an amount of light energy received by the observation target OB or a living body in the vicinity (there is a risk of causing a burn when the amount of light energy is large); or a fading speed of observation target fluorescence emitted from an agent. The light amount of the excitation light can also be adjusted by changing the number of light sources of the excitation light mounted on the light source device 3. However, the number of light sources may influence the size of the light source device 3. The size of the light source device 3 may be limited by the size of a cart for carrying the light source device 3 or the like.

That is, it is difficult to adjust the light amount of the observation target fluorescence by adjusting the light amount of the excitation light. As a result, it is difficult to adjust the signal value based on the observation target fluorescence imaged by the image sensor 513 by adjusting the light amount of the excitation light.

(4) Image sensor

The signal value based on the observation target fluorescence generated from the image sensor 513 differs depending on the light amount of the observation target fluorescence received by the image sensor 513.

In order to adjust the signal value based on the observation target fluorescence, it is desired to select the image sensor 513 having optimum sensitivity, configuration, and the like for imaging the observation target fluorescence. However, the image sensor 513 might be required not only to output an image for fluorescence observation based on reception of observation target fluorescence in a predetermined wavelength band, but also to output an image for normal light observation based on reception of visible light such as white light. In addition, the image sensor 513 might also be required to output an image for fluorescence observation corresponding to a wide wavelength band or a plurality of wavelength bands among wavelength bands including visible light and invisible light. Furthermore, even if the same agent is used, the light amount of the observation target fluorescence might change depending on the procedure or the observation target OB. Therefore, the image sensor 513 might also be required to output an image for fluorescence observation corresponding to such a change in the light amount of the observation target fluorescence. In that case, the image sensor 513 needs to select a sensor element capable of supporting these observations, leading to a case of not being able to use an image sensor having optimum characteristics for imaging fluorescence in a predetermined wavelength band. In addition, the image sensor 513 is disposed in the camera head 5, and since the camera head 5 has a size and weight suitable for observation, the type including the size of the image sensor 513 might be limited. Not only in the configuration in which the image sensor 513 is disposed in the camera head 5, but also in a case where the image sensor 513 is disposed at the distal end of a rigid endoscope or a flexible endoscope, the image sensor 513 has a size and weight suitable for observation, leading to the limitation of the type including the size of the image sensor 513.

That is, it is difficult to adjust the signal value based on the observation target fluorescence imaged by the image sensor 513 by selecting the type of the image sensor 513.

As described above, the signal value based on the observation target fluorescence captured by the image sensor 513 is determined within the above constraint, and thus cannot be easily adjusted.

In the present embodiment, the observation target fluorescence has a spectral characteristic of a wavelength band having a center wavelength of about 650[nm] as illustrated in a spectrum SPF1 of FIG. 4. That is, in the present embodiment, the observation target fluorescence is light in a wavelength band of visible light. In addition, the unnecessary light has a spectral characteristic of a wavelength band over the entire wavelength band of the visible light and the wavelength band of the invisible light as illustrated in a wavelength range SPU of FIG. 4 in which the unnecessary light can be generated. The spectral characteristic of light such as the observation target fluorescence and unnecessary light is also referred to as a radiation spectral characteristic. In the present embodiment, the spectral characteristics of the observation target fluorescence and unnecessary light are illustrated in FIG. 4. However, the spectral characteristics can vary depending on the type and concentration of the fluorescent agent, the wavelength characteristics and intensity of the excitation light, the type and amount of the member forming the optical path, etc. That is, the spectral characteristics of the observation target fluorescence and unnecessary light may be other spectral characteristics, not limited to those illustrated in FIG. 4.

Accordingly, from the spectral characteristics of the R filter group 514r and the spectral characteristics of the observation target fluorescence and the unnecessary light, the signal value of the pixel signal corresponding to the pixel corresponding to the R filter group 514r (hereinafter, denoted as an r-value) includes a component of the observation target fluorescence and a component of the unnecessary light. In addition, from the spectral characteristics of the G filter group 514g and the spectral characteristics of the observation target fluorescence and the unnecessary light, the signal value of the pixel signal corresponding to the pixel corresponding to the G filter group 514g (hereinafter, denoted as a g-value) mainly includes the component of the unnecessary light. Furthermore, from the spectral characteristics of the B filter group 514b and the spectral characteristics of the observation target fluorescence and the unnecessary light, the signal value of the pixel signal corresponding to the pixel corresponding to the B filter group 514b (hereinafter, denoted as a b-value) mainly includes the component of the unnecessary light in a similar manner. That is, the r-value includes a larger quantity of component of the observation target fluorescence in comparison with the g-value and the b-value.

As described above, the r-value, the g-value, and the b-value include a component of unnecessary light. In order to satisfactorily perform fluorescence observation, the present embodiment performs image processing (correction matrix processing) executed by the control device 9 described below, thereby generating a corrected pixel signal that has suppressed a component of unnecessary light to become noise in performing fluorescence observation (which is a signal that has reduced a signal value based on the unnecessary light).

Note that each filter group constituting the color filter 514 is not limited to the filter groups 514r, 514g, and 514b that mainly transmit light in the R, G, and B wavelength bands, respectively. That is, the color filter 514 is not limited to the color filter having the above-described spectral characteristics, and may be configured by a plurality of filter groups each having other spectral characteristics.

[Configuration of control device]

Next, the configuration of the control device 9 will be described with reference to FIG. 2.

As illustrated in FIG. 2, the control device 9 includes a communication unit 91, an image processing unit 92, a control unit 93, an input unit 94, an output unit 95, and a storage unit 96.

The communication unit 91 functions as a receiver that receives captured images sequentially transmitted from the camera head 5 (communication unit 52) via the first transmission cable 6. For example, the communication unit 91 includes a high-speed serial interface that performs captured image communication with the communication unit 52 at a transmission rate of 1 Gbps or more. Incidentally, the communication unit 91 may perform communication at a transmission rate lower than 1 Gbps, for example, in a case where the image sensor 513 is an image sensor with HD resolution or lower.

Under the control of the control unit 93, the image processing unit 92 processes the captured image sequentially output from the camera head 5 (communication unit 52) and received by the communication unit 91. As illustrated in FIG. 2, the image processing unit 92 includes a WB processing unit 921, a demosaic processing unit 922, a correction matrix processing unit 923, a gamma processing unit 924, and a YC converter 925.

The WB processing unit 921 performs white balance (WB) processing of multiplying each of the r-value, the g-value, and the b-value in the captured image by a specific gain. Hereinafter, the r-value, the g-value, and the b-value in the captured image output from the WB processing unit 921 are denoted by $R_{921}$, $G_{921}$, and $B_{921}$, respectively (FIG. 2).

The demosaic processing unit 922 performs demosaic processing of assigning, by interpolation, signal values of an r-value, a g-value, and a b-value ($R_{922}$, $G_{922}$, and $B_{922}$) to each pixel on the captured image that has undergone WB processing.

Here, in the present embodiment, as described above, from the spectral characteristics of the color filter 514 and the spectral characteristics of the observation target fluorescence and unnecessary light, the r-value has a larger quantity of component of the observation target fluorescence in comparison with the g-value and the b-value. Accordingly, also in the signal value ($R_{922}$, $G_{922}$, and $B_{922}$) after the demosaic processing, the signal value ($R_{922}$) has a larger quantity of component of the observation target fluorescence in comparison with the signal values ($G_{922}$ and $B_{922}$). Accordingly, the signal value ($R_{922}$) corresponds to a first pixel signal (signal value) according to the present disclosure. In addition, the signal values ($G_{922}$ and $B_{922}$) correspond to a second pixel signal (signal value) according to the present disclosure. The signal value ($R_{922}$) is a signal value in which the component of the observation target fluorescence is larger than the component of the unnecessary light, and the component of the observation target fluorescence is dominant. On the other hand, the signal values ($G_{922}$ and $B_{922}$) are signal values in each of which a component of unnecessary light is dominant.

In the WB processing described above, by multiplying the r-value, the g-value, and the b-value individually by specific gains, processing is performed such that the components of the unnecessary light included in the r-value, the g-value, and the b-value are substantially the same (processing of adjusting the magnitude relationship of the components of the unnecessary light). In other words, in the WB processing, processing is performed such that the components of the unnecessary light included in the signal value ($R_{922}$), the signal value ($G_{922}$), and the signal value ($B_{922}$) are substantially the same based on the spectral characteristic of the color filter 514. Accordingly, the WB processing unit 921 corresponds to a spectral sensitivity correction unit according to the present disclosure. The WB processing corresponds to the spectral sensitivity correction processing according to the present disclosure.

The correction matrix processing unit 923 corresponds to a signal processing unit according to the present disclosure. The correction matrix processing unit 923 generates a corrected pixel signal based on the correction coefficient and the signal values ($R_{922}$, $G_{922}$, and $B_{922}$).

In the present embodiment, the correction matrix processing unit 923 performs correction matrix processing of generating a corrected pixel signal by multiplying a correction matrix $M_C$, which is a correction coefficient according to the present disclosure, by an input matrix $M_{922}$ each including signal values ($R_{922}$, $G_{922}$, and $B_{922}$) as matrix elements.

Specifically, in the correction matrix processing, the correction matrix processing unit 923 corrects the signal values ($R_{922}$, $G_{922}$, and $B_{922}$) for each pixel to signal values ($R_{923}$, $G_{923}$, and $B_{923}$) by the following Formula (1). The signal values ($R_{923}$, $G_{923}$, and $B_{923}$) correspond to the signal values of the corrected pixel signal.

$$\begin{pmatrix} R_{923} \\ G_{923} \\ B_{923} \end{pmatrix} = M_C * M_{922}$$

$$M_C = \begin{pmatrix} a & b & c \\ d & e & f \\ g & h & i \end{pmatrix}$$

$$M_{922} = \begin{pmatrix} R_{922} \\ G_{922} \\ B_{922} \end{pmatrix} \qquad \cdot \cdot \cdot \ (1)$$

Details of the correction matrix $M_C$ will be described in "Correction matrix $M_C$" described below.

The gamma processing unit 924 performs gamma processing ($\gamma$ correction) on the captured image (the signal values for each pixel ($R_{923}$, $G_{923}$, and $B_{923}$)) that has undergone the correction matrix processing.

The YC converter 925 performs YC conversion for converting the captured image (RGB signal) that has undergone the gamma processing into a luminance signal and a color difference signal ($Y,C_B/C_R$ signal). Subsequently, the image processing unit 92 outputs the captured image that has undergone the YC conversion to the display device 7 via the second transmission cable 8. With this operation, the display device 7 displays the captured image.

Note that the image processing unit 92 is not limited to the configuration of performing the demosaic processing between the WB processing and the correction matrix processing. The demosaic processing may be performed at any timing as long as the demosaic processing is performed before the gamma processing. That is, as the image processing unit 92, the processing units 921 to 925 may be arranged in an order different from the order illustrated in FIG. 2.

The control unit 93 is implemented by executing various programs stored in the storage unit 96 by a controller such as a CPU or a micro processing unit (MPU). The control unit 93 controls the operations of the light source device 3, the camera head 5, and the display device 7 and controls the entire operation of the control device 9. The control unit 93 may include an integrated circuit such as an application specific integrated circuit (ASIC) or an FPGA, not limited to the CPU or the MPU.

The input unit 94 includes an operation device such as a mouse, a keyboard, and a touch panel, and receives user operations performed by a user such as a practitioner. Subsequently, the input unit 94 outputs an operation signal corresponding to the user operation to the control unit 93.

The output unit 95 includes a speaker, a printer, or the like, and outputs various types of information.

The storage unit 96 stores a program executed by the control unit 93, information needed for processing performed by the control unit 93, or the like.

[Correction matrix $M_C$]

Next, the correction matrix $M_C$ will be described.

The correction matrix $M_C$ is set based on at least the spectral characteristic of the observation target fluorescence. In the present embodiment, the correction matrix $M_C$ is set based on the spectral characteristic of the observation target fluorescence, the spectral characteristic of the unnecessary light, and the spectral characteristic of the color filter 514.

Specifically, the matrix element multiplied by the first pixel signal (signal value) in the correction matrix $M_C$ is set to 0 or a positive value. In the present embodiment, as described above, the first pixel signal (signal value) is the signal value ($R_{922}$), which is a signal value having a relatively large quantity of component of the observation target fluorescence. Accordingly, "a", "d", and "g", which are matrix elements (refer to Formula (1)) multiplied by the signal value ($R_{922}$) in the correction matrix $M_C$, are set to 0 or positive values.

On the other hand, the matrix element multiplied by the second pixel signal (signal value) in the correction matrix $M_C$ is set to 0 or a negative value. In the present embodiment, as described above, the second pixel signal (signal value) corresponds to the signal values ($G_{922}$, and $B_{922}$) and is a signal value mainly including a component of unnecessary light. Accordingly, "b", "c", "e", "f", "h", and" i", which are matrix elements (refer to Formula (1)) multiplied by the signal values

($G_{922}$ and $B_{922}$) in the correction matrix $M_C$, are set to 0 or negative values.

Furthermore, it is conceivable to set the matrix elements in the correction matrix $M_C$ by the following first to third setting methods.

The first setting method is a setting method of setting the sum of matrix elements to 0 in each row of the correction matrix $M_C$ such that (a + b + c = d + e + f = g + h + i = 0). In the case of the present embodiment, the correction matrix $M_C$ set by the first setting method can be exemplified by a correction matrix of the following Formula (2).

$$M_C = \begin{pmatrix} 1 & -1 & 0 \\ 1 & 0 & -1 \\ 0 & 0 & 0 \end{pmatrix} \qquad \cdot \cdot \cdot \ (2)$$

The second setting method is a setting method of setting the sum of matrix elements so as to be each larger than 0 in each row of the correction matrix $M_C$ such that (a + b + c = d + e + f = g + h + i > 0).

The third setting method is a setting method of setting the sum of matrix elements so as to be each smaller than 0 in each row of the correction matrix $M_C$ such that (a + b + c = d + e + f = g + h + i < 0).

In the correction matrix $M_C$, the ratio or the like of "a", "d", and "g" in the first column, "b", "e", and "h" in the second column, and "c", "f", and "i" in the third column may be set in consideration of the spectral sensitivity ratio of each of the filter groups 514r, 514g, and 514b.

In addition, when it is desired to enhance the observation fluorescence component or when it is desired to express the observation fluorescence component as a pseudo color, it is also allowable, in the correction matrix $M_C$, to multiply "a", "b", and "c" in the first row, "d", "e", and" f" in the second row, and "g", "h", and "i" in the third row by mutually different gain factors.

The present embodiment described above achieves the following effects.

In the control device 9 according to the present embodiment, the correction matrix processing unit 923 generates the corrected pixel signal (signal values ($R_{923}$, $G_{923}$, and $B_{923}$) in the present embodiment) based on the correction coefficient (the correction matrix $M_C$ in the present embodiment), the first pixel signal (the signal value ($R_{922}$) in the present embodiment) in which the component of the observation target fluorescence is dominant, and the second pixel signals (signal values ($G_{922}$ and $B_{922}$) in the present embodiment) in which the component of the unnecessary light is dominant.

Therefore, according to the control device 9 of the present embodiment, the second pixel signal in which the component of the unnecessary light is dominant is subtracted from the first pixel signal in which the component of the observation target fluorescence is dominant, making it possible to generate the corrected pixel signal that has suppressed the component of unnecessary light that becomes noise in performing the fluorescence observation. That is, fluorescence observation can be performed satisfactorily.

In particular, the correction matrix processing unit 923 generates the corrected pixel signals (signal values ($R_{923}$, $G_{923}$, and $B_{923}$) in the present embodiment)) by multiplying a correction matrix $M_C$, which is a correction coefficient according to the present disclosure, by the input matrix $M_{922}$ each including the first and second pixel signals (signal values $R_{922}$, $G_{922}$, and $B_{922}$ in the present embodiment) as matrix elements.

Accordingly, the corrected pixel signal can be easily generated by simple processing.

The matrix element multiplied by the signal value of the first pixel signal in the correction matrix $M_C$ is set to 0 or a positive value, and the matrix element multiplied by the signal value of the second pixel signal is set to 0 or a negative value. Furthermore, by setting the matrix elements by the first to third setting methods, the following effects can be obtained.

In a case where the matrix elements in the correction matrix $M_C$ are set by the first setting method, it is possible to generate a corrected pixel signal that has substantially removed or suppressed the unnecessary light component. In a case where the matrix elements in the correction matrix $M_C$ are set by the second setting method, it is possible to generate a corrected pixel signal that has substantially removed or suppressed only a part of the unnecessary light component. In a case where the matrix elements in the correction matrix $M_C$ are set by the third setting method, it is possible to generate a corrected pixel signal that contains variations and that has substantially removed or suppressed the unnecessary light component.

In addition, the control device 9 includes the WB processing unit 921 that executes WB processing of making the component of unnecessary light included in the first pixel signal and the component of unnecessary light included in the second pixel signal substantially the same. The correction matrix processing unit 923 generates the corrected pixel signal by using the first and second pixel signals (signal values ($R_{922}$, $G_{922}$, and $B_{922}$) in the present embodiment) that have undergone the WB processing.

Accordingly, by subtracting the second pixel signal in which the component of the unnecessary light is dominant from the first pixel signal in which the component of the observation target fluorescence is dominant, it is possible to generate a corrected pixel signal that has efficiently suppressed the component of the unnecessary light.

(Other embodiments)

While the above is description of the modes for carrying out the present disclosure, the present disclosure should not be limited by only the embodiment described above.

In the above-described embodiment, the light source device 3 emits only the excitation light. However, the present invention is not limited thereto, and it is also allowable to adopt a configuration that emits white light in addition to the excitation light. In this case, the camera head 5 generates a captured image (hereinafter, referred to as a fluorescence image) obtained by capturing the observation target fluorescence generated in the observation target OB in response to the excitation light and a captured image (hereinafter, referred to as a white light image) obtained by capturing the white light reflected from the observation target OB. In addition, the control device 9 superimposes the fluorescence image on the white light image, displays the white light image and the fluorescence image in parallel, displays the white light image and the fluorescence image as a PinP display, or displays the white light image and the fluorescence image on mutually different monitors.

Even in such a configuration, the effects similar to those of the above-described embodiment can be obtained.

In the above-described embodiment, the following first to tenth modifications may be adopted.

(First modification)

A first modification will describe a case of using the ALM-488 as an agent.

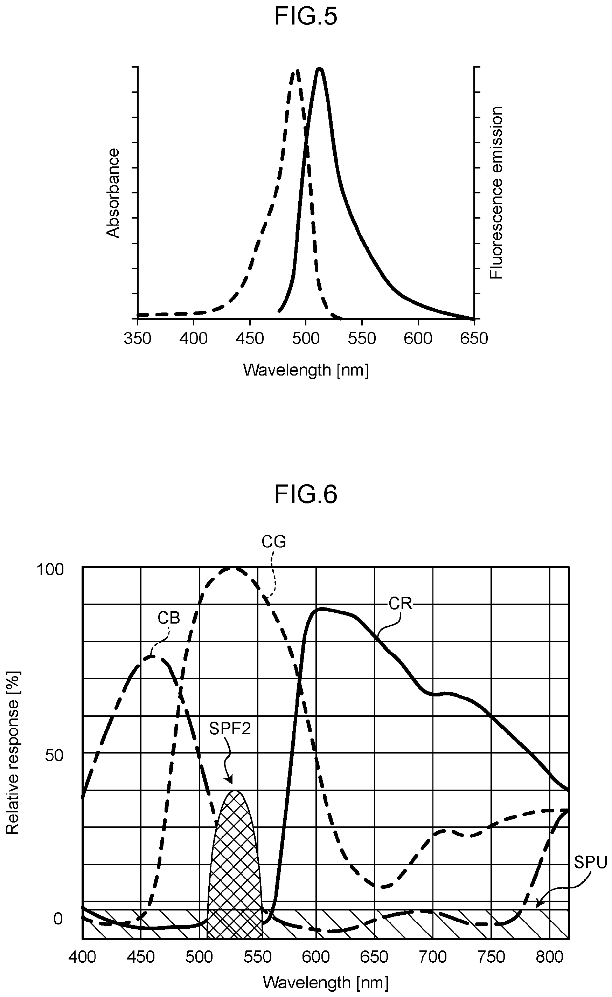

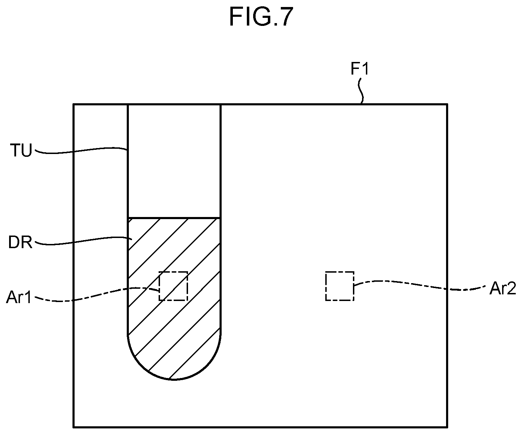

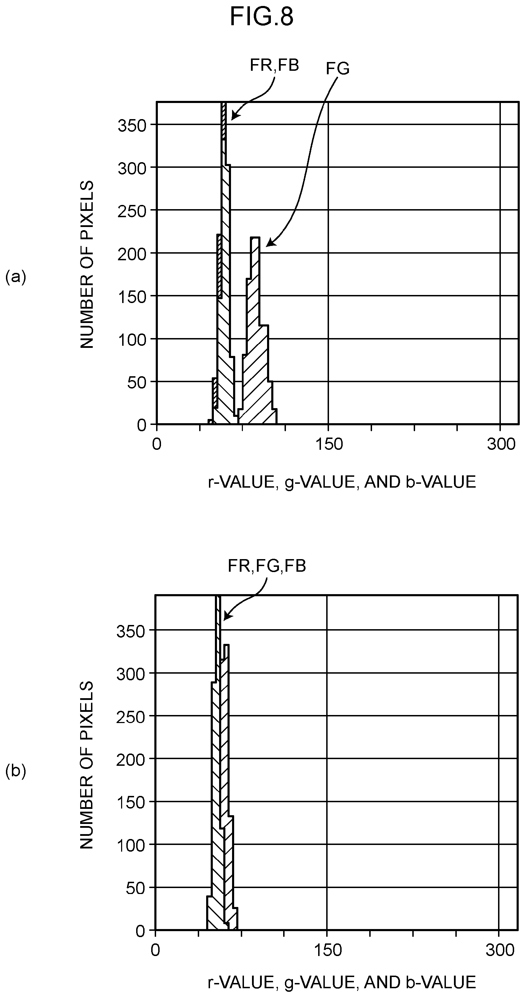

FIGS. 5 to 8 are diagrams illustrating the first modification of the embodiment. Specifically, FIG. 5 is a diagram illustrating an absorption spectrum and an emission spectrum of the ALM-488. In FIG. 5, the horizontal axis represents Wavelength[nm], and the vertical axis represents Absorbance of the absorption spectrum and Fluorescence emission of the emission spectrum. In FIG. 5, the absorption spectrum of the ALM-488 is indicated by a broken line, and the emission spectrum of the ALM-488 is indicated by a solid line. FIG. 6 is a diagram corresponding to FIG. 4. FIG. 7 illustrates a captured image F1 obtained by capturing an area including an agent DR as a subject in a state where a test tube TU containing the agent DR of the ALM-488 is arranged in front of a blackout curtain and the agent DR is irradiated with excitation light. (a) of FIG. 8 is a diagram illustrating frequency distributions of r-values, g-values, and b-values in a first area Ar1 in the captured image F1 illustrated in FIG. 7. (b) of FIG. 8 is a diagram illustrating frequency distributions of r-values, g-values, and b-values of a second area Ar2 in the captured image F1 illustrated in FIG. 7. In FIG. 8, the horizontal axis represents the r-value, the g-value, and the b-value, and the vertical axis represents the number of pixels (frequency) of the r-value, the g-value, and the b-value.

The peak wavelength of the absorption spectrum of the ALM-488 is about 480[nm] as illustrated in FIG. 5. Accordingly, the excitation light emitted from the light source device 3 is preferably excitation light in a wavelength band including a wavelength of about 480[nm] in order to increase the intensity of the observation target fluorescence emitted from the ALM-488.

In addition, the peak wavelength of the emission spectrum of the ALM-488 is about 530[nm] as illustrated in FIG. 5. Accordingly, the observation target fluorescence emitted from the ALM-488 has a spectral characteristic of a wavelength band having a center wavelength of about 530[nm] as illustrated in a spectrum SPF2 of FIG. 6. From the spectral characteristics of the G filter group 514g and the spectral characteristics of the observation target fluorescence and the unnecessary light, the g-value includes the component of the observation target fluorescence and the component of the unnecessary light. In addition, from the spectral characteristics of the R filter group 514r and the spectral characteristics of the observation target fluorescence and the unnecessary light, the r-value mainly includes the component of the unnecessary light. Furthermore, from the spectral characteristics of the B filter group 514b and the spectral characteristics of the observation target fluorescence and the unnecessary light, the b-value mainly includes the component of the unnecessary light in a similar manner. That is, the g-value includes a larger quantity of component of the observation target fluorescence in comparison with the r-value and the b-value.

The following experiment has also revealed that "In the case of using the ALM-488, there is a larger quantity of component of the observation target fluorescence contained in the g-value, in comparison with the r-value and the b-value".

In this experiment, the test tube TU containing the agent DR of the ALM-488 is arranged in front of the blackout curtain, and the agent DR is irradiated with excitation light. In this state, the imaging unit 51 was caused to capture an area including the agent DR as a subject, and frequency distributions of r-values, g-values, and b-values in the first and second areas Ar1 and Ar2 in the captured image F1 generated by the image capturing were compared with each other (FIG. 8). Here, as illustrated in FIG. 7, the first area Ar1 is an area in which the agent DR in the captured image F1 appears. In contrast, the second area Ar2 is an area of a background (blackout curtain) other than the agent DR in the captured image F1. That is, since the observation target fluorescence is emitted from the agent DR by the irradiation of the excitation light, the first area Ar1 is brighter than the second area Ar2.

In FIG. 8, a reference sign "FR" denotes a frequency distribution of r-values. A reference sign "FG" denotes a frequency distribution of g-values. A reference sign "FB" denotes a frequency distribution of the b-value.

As a result, as illustrated in (a) of FIG. 8, the frequency distribution FG of the g-value appears in the bright portion (the portion where the value of the horizontal axis of FIG. 8 is large), and the frequency distributions FR and FB of the r-value and the b-value appear in the portion darker than the frequency distribution FG (the portion where the value of the horizontal axis of FIG. 8 is small). This has revealed that "In the case of using the ALM-488, there is a larger quantity of component of the observation target fluorescence contained in the g-value, in comparison with the r-value and the b-value". Note that the frequency distributions FR and FB of the r-value and the b-value in the first area Ar1 illustrated in (a) of FIG. 8 and the frequency distributions FR, FG, and FB of the r-value, the g-value, and the b-value in the second area Ar2 illustrated in (b) of FIG. 8 mainly include the components of unnecessary light.

As described above, the g-value has a larger quantity of component of the observation target fluorescence in comparison with the r-value and the b-value, and thus, also the signal values ($R_{922}$, $G_{922}$, and $B_{922}$), the signal value ($G_{922}$) has a larger quantity of component of the observation target fluorescence in comparison with the signal values ($R_{922}$ and $B_{922}$). Accordingly, the signal value ($G_{922}$) corresponds to the first pixel signal (signal value) according to the present disclosure. In addition, the signal values ($R_{922}$ and $B_{922}$) correspond to the second pixel signals (signal values) according to the present disclosure. The signal value ($G_{922}$) is a signal value in which the component of the observation target fluorescence is larger than the component of the unnecessary light, and the component of the observation target fluorescence is dominant. On the other hand, the signal values ($R_{922}$ and $B_{922}$) are signal values in each of which a component of unnecessary light is dominant.

Also in the case of using the ALM-488, the matrix element multiplied by the first pixel signal (signal value) in the correction matrix $M_C$ is set to 0 or a positive value, similarly to the above-described embodiment. Specifically, in the case of using the ALM-488, as described above, the first pixel signal (signal value) is the signal value ($G_{922}$) having a relatively large quantity of component of the observation target fluorescence. Accordingly, "b", "e", and "h", which are matrix elements (refer to Formula (1)) multiplied by the signal value ($G_{922}$) in the correction matrix $M_C$, are set to 0 or positive values.

Also in the case of using the ALM-488, the matrix element multiplied by the second pixel signal (signal value) in the correction matrix $M_C$ is set to 0 or a negative value, similarly to the above-described embodiment. Specifically, in the case of using the ALM-488, as described above, the second pixel signal (signal value) is a signal value ($R_{922}$ and $B_{922}$) mainly including the component of unnecessary light. Accordingly, the matrix elements "a", "d", "g", "c", "f", and "i" multiplied by the signal value ($R_{922}$ and $B_{922}$) in the correction matrix $M_C$ (refer to Formula (1)) are set to 0 or negative values.

The correction matrix $M_C$ in the case of using the ALM-488 can be set by the first to third setting methods similarly to the above-described embodiment. In the case of using the ALM-488, the correction matrix $M_C$ set by the first setting method can be exemplified by a correction matrix of the following Formula (3).

$$M_C = \begin{pmatrix} -1 & 1 & 0 \\ 0 & 1 & -1 \\ 0 & 0 & 0 \end{pmatrix} \qquad \cdots \; (3)$$

Even in the case of using the ALM-488 as the agent as in the first modification described above, the effects similar to those of the above-described embodiment can be obtained.

(Second modification)

A second modification will describe a case of using LUM-015 as an agent.

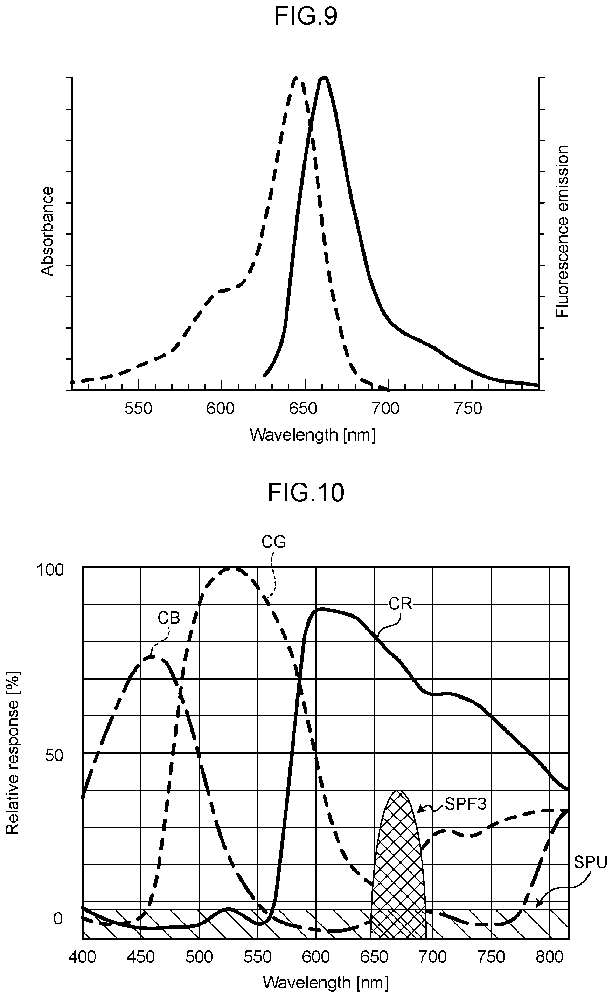

FIGS. 9 and 10 are diagrams each illustrating the second modification of the embodiment. Specifically, FIG. 9 is a diagram illustrating an absorption spectrum and an emission spectrum of LUM-015. In FIG. 9, the horizontal axis represents Wavelength [nm], and the vertical axis represents Absorbance of the absorption spectrum and Fluorescence emission of the emission spectrum. In FIG. 9, the absorption spectrum of LUM-015 is indicated by a broken line, the emission spectrum of LUM-015 is indicated by a solid line, and FIG. 10 is a diagram corresponding to FIG. 4.

The peak wavelength of the absorption spectrum of LUM-015 is about 650[nm] as illustrated in FIG. 9. Accordingly, the excitation light emitted from the light source device 3 is preferably excitation light in a wavelength band including a wavelength of about 650[nm] in order to increase the intensity of the observation target fluorescence emitted from the LUM-015.

The peak wavelength of the emission spectrum of LUM-015 is about 670[nm] as illustrated in FIG. 9. Accordingly, the observation target fluorescence emitted from LUM-015 has a spectral characteristic of a wavelength band having a center wavelength of about 670[nm] as illustrated in a spectrum SPF3 of FIG. 10. From the spectral characteristics of the R filter group 514r and the spectral characteristics of the observation target fluorescence and the unnecessary light, the r-value includes the component of the observation target fluorescence and the component of the unnecessary light. In addition, from the spectral characteristics of the G filter group 514g and the spectral characteristics of the observation target fluorescence and the unnecessary light, the g-value mainly includes the component of the unnecessary light. Furthermore, from the spectral characteristics of the B filter group 514b and the spectral characteristics of the observation target fluorescence and the unnecessary light, the b-value mainly includes the component of the unnecessary light in a similar manner. That is, the r-value includes a larger quantity of component of the observation target fluorescence in comparison with the g-value and the b-value.

As described above, the r-value has a larger quantity of component of the observation target fluorescence in comparison with the g-value and the b-value, and thus, also in the signal values ($R_{922}$, $G_{922}$, and $B_{922}$), the signal value ($R_{922}$) has a larger quantity of component of the observation target fluorescence in comparison with the signal values ($G_{922}$ and $B_{922}$). Accordingly, the signal value ($R_{922}$) corresponds to a first pixel signal (signal value) according to the present disclosure. In addition, the signal values ($G_{922}$ and $B_{922}$) correspond to a second pixel signal (signal value) according to the present disclosure. The signal value ($R_{922}$) is a signal value in which the component of the observation target fluorescence is larger than the component of the unnecessary light, and the component of the observation target fluorescence is dominant. On the other hand, the signal values ($G_{922}$ and $B_{922}$) are signal values in each of which a component of unnecessary light is dominant.

That is, similarly to the above-described embodiment, when LUM-015 is used, the signal value ($R_{922}$) corresponds to the first pixel signal (signal value) according to the present disclosure, and the signal values ($G_{922}$ and $B_{922}$) correspond to the second pixel signals (signal values) according to the present disclosure. Accordingly, the correction matrix described in the above-described embodiment can be used as the correction matrix $M_C$ in the case of using LUM-015.

Even in a case of using LUM-015 as the agent as in the second modification described above, the effects similar to those of the above-described embodiment can be obtained.

(Third modification)

A third modification will describe a case of using a plurality of agents.

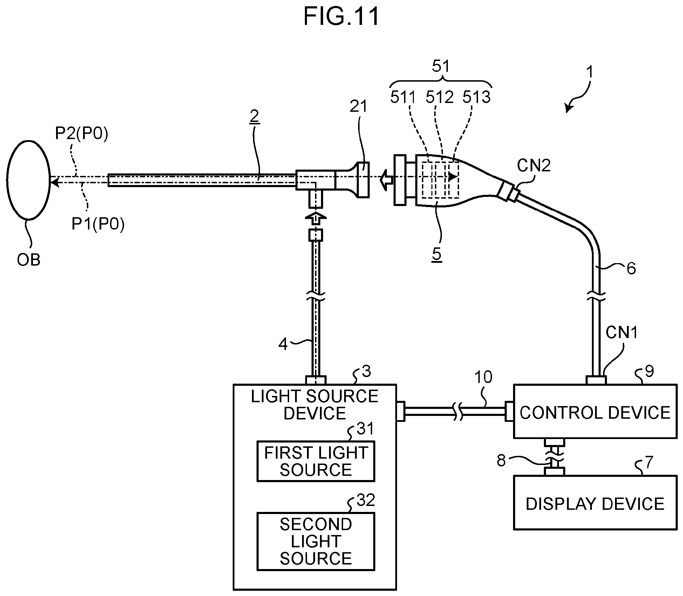

FIG. 11 is a diagram illustrating the third modification of the embodiment. Specifically, FIG. 11 is a diagram corresponding to FIG. 1. FIG. 11 illustrates the medical observation system 1 in a case where the number of agents to be used is two. Hereinafter, for convenience of description, the number of agents to be used is two.

In a case where the number of agents to be used is two, as illustrated in FIG. 11, there is a need to have a configuration of the medical observation system 1 including a second light source 32 that emits excitation light for exciting a second agent in addition to the first light source 31 that emits excitation light for exciting the first agent among the two agents. When the number of agents to be used is three or more, three or more light sources may be provided corresponding to the number of agents.

The medical observation system 1 turns on the first and second light sources 31 and 32 in a time-division manner for the observation target OB to which the first and second agents are applied. The medical observation system 1 uses the correction matrix $M_C$ corresponding to the first agent when executing correction matrix processing on the captured image generated by imaging by the image sensor 513 at the lighting timing of the first light source 31. On the other hand, the medical observation system 1 uses the correction matrix $M_C$ corresponding to the second agent when executing correction matrix processing on the captured image generated by imaging by the image sensor 513 at the lighting timing of the second light source 32.

Even in the case of using a plurality of agents as in the third modification described above, the effects similar to those of the above-described embodiment can be obtained.

(Fourth modification)

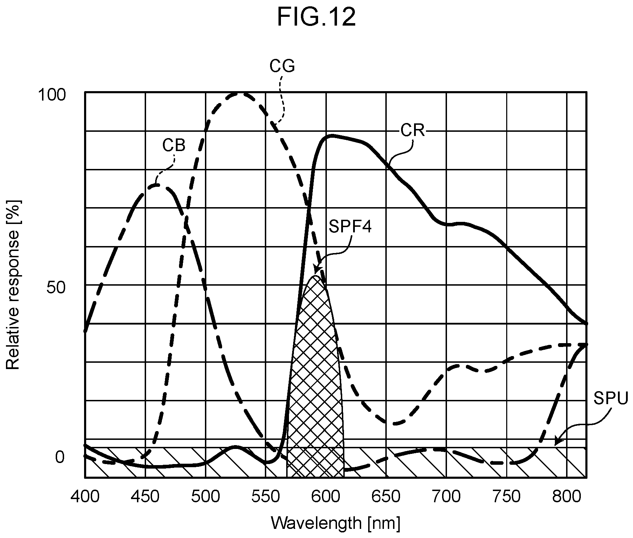

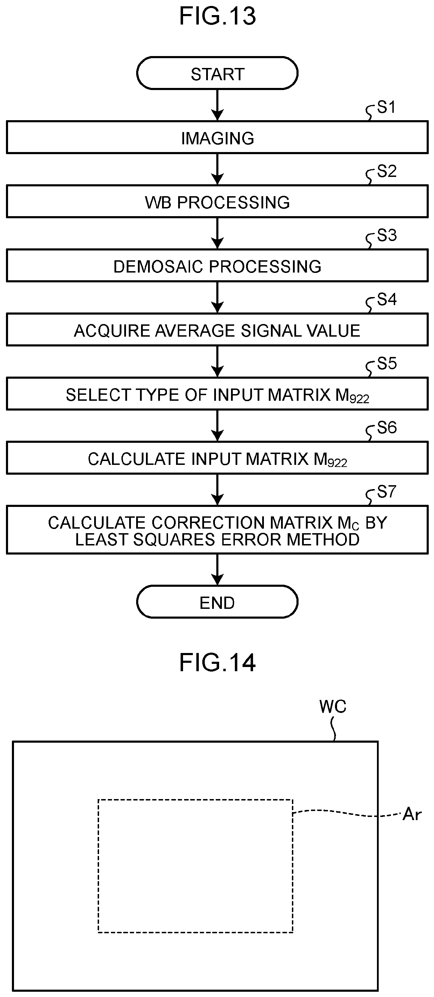

FIG. 12 to 14 are diagrams illustrating a fourth modification of the embodiment. Specifically, FIG. 12 is a diagram corresponding to FIG. 4. FIG. 13 is a flowchart illustrating a method of calculating the correction matrix $M_C$. FIG. 14 is a diagram illustrating a white chart WC used in the method of calculating the correction matrix $M_C$ illustrated in FIG. 13.

While the above-described embodiment uses the exemplary case where the matrix of 3*3 is adopted as the correction matrix $M_C$, the matrix is not limited thereto. For example, it is also allowable to adopt other matrices, such as a correction matrix $M_C$ of 3*8 expressed by the following Formula (4), a correction matrix $M_C$ of 3*7 expressed by the following Formula (5), a correction matrix $M_C$ of 3*6 expressed by the following Formula (6), a correction matrix $M_C$ of 3*5 expressed by the following Formula (7), or a correction matrix $M_C$ of 3*4 expressed by the following Formula (8).

$$M_C = \begin{pmatrix} a & b & c & d & e & f & g & h \\ i & j & k & l & m & n & o & p \\ q & r & s & t & u & v & w & x \end{pmatrix}$$

$$M_{922} = \begin{pmatrix} R_{922} \\ G_{922} \\ B_{922} \\ R_{922} * G_{922} \\ G_{922} * B_{922} \\ R_{922} * B_{922} \\ R_{922} * G_{922} * B_{922} \\ 1 \end{pmatrix} \qquad \cdots (4)$$

$$M_C = \begin{pmatrix} a & b & c & d & e & f & g \\ h & i & j & k & l & m & n \\ o & p & q & r & s & t & u \end{pmatrix}$$

$$M_{922} = \begin{pmatrix} R_{922} \\ G_{922} \\ B_{922} \\ R_{922} * G_{922} \\ G_{922} * B_{922} \\ R_{922} * B_{922} \\ 1 \end{pmatrix} \qquad \cdots (5)$$

$$M_C = \begin{pmatrix} a & b & c & d & e & f \\ g & h & i & j & k & l \\ m & n & o & p & q & r \end{pmatrix}$$

$$M_{922} = \begin{pmatrix} R_{922} \\ G_{922} \\ B_{922} \\ R_{922} * G_{922} \\ G_{922} * B_{922} \\ 1 \end{pmatrix}$$

$$M_{922} = \begin{pmatrix} R_{922} \\ G_{922} \\ B_{922} \\ R_{922} * G_{922} \\ R_{922} * B_{922} \\ 1 \end{pmatrix}$$

$$M_{922} = \begin{pmatrix} R_{922} \\ G_{922} \\ B_{922} \\ G_{922} * B_{922} \\ R_{922} * B_{922} \\ 1 \end{pmatrix} \qquad \cdots (6)$$

$$M_C = \begin{pmatrix} a & b & c & d & e \\ f & g & h & i & j \\ k & l & m & n & o \end{pmatrix}$$

$$M_{922} = \begin{pmatrix} R_{922} \\ G_{922} \\ B_{922} \\ R_{922} * G_{922} \\ 1 \end{pmatrix}$$

$$M_{922} = \begin{pmatrix} R_{922} \\ G_{922} \\ B_{922} \\ G_{922} * B_{922} \\ 1 \end{pmatrix}$$

$$M_{922} = \begin{pmatrix} R_{922} \\ G_{922} \\ B_{922} \\ R_{922} * B_{922} \\ 1 \end{pmatrix} \quad \cdots \ (7)$$

$$M_C = \begin{pmatrix} a & b & c & d \\ e & f & g & h \\ i & j & k & l \end{pmatrix}$$

$$M_{922} = \begin{pmatrix} R_{922} \\ G_{922} \\ B_{922} \\ 1 \end{pmatrix} \quad \cdots \ (8)$$

The input matrix $M_{922}$ indicated in Formula (4) includes signal values ($R_{922}$, $G_{922}$, and $B_{922}$) as matrix elements, and also includes, as matrix elements, "$R_{922}*G_{922}$" obtained by multiplying the signal value ($R_{922}$) and the signal value ($G_{922}$), "$G_{922}*B_{922}$" obtained by multiplying the signal value ($G_{922}$) and the signal value ($B_{922}$), "$R_{922}*B_{922}$" obtained by multiplying the signal value ($R_{922}$) and the signal value ($B_{922}$), "$R_{922}*G_{922}*B_{922}$" obtained by multiplying the signal value ($R_{922}$), the signal value ($G_{922}$), and the signal value ($B_{922}$), and "1".

The input matrix $M_{922}$ indicated in Formula (5) is obtained by omitting the matrix element of "$R_{922}*G_{922}*B_{922}$" in the input matrix $M_{922}$ indicated in Formula (4).

Although Formula (6) indicates three input matrices $M_{922}$, any one of the three input matrices $M_{922}$ may be used. The three input matrices $M_{922}$ are obtained by omitting any one matrix element among "$R_{922}*G_{922}$", "$G_{922}*B_{922}$", and "$R_{922}*B_{922}$" in the input matrix $M_{922}$ indicated in Formula (5).

Although Formula (7) indicates three input matrixes $M_{922}$, any one of the three input matrices $M_{922}$ may be used. The three input matrices $M_{922}$ are obtained by omitting any two matrix elements among "$R_{922}*G_{922}$", "$G_{922}*B_{922}$", and "$R_{922}*B_{922}$" in the input matrix $M_{922}$ indicated in Formula (5).

The input matrix $M_{922}$ indicated in Formula (8) is obtained by adding a matrix element of "1" to the input matrix $M_{922}$ indicated in Formula (1).

Meanwhile, the input matrix $M_{922}$ preferably includes, as matrix elements, a signal value in which the component of the observation target fluorescence is dominant and a signal value in which the component of the unnecessary light is dominant. For example, as illustrated in the spectrum SPF4 of FIG. 12, in a case where the spectral characteristic of the observation target fluorescence is a spectral characteristic of a wavelength band having a center wavelength of about 580 [nm], the signal value ($R_{922}$) and the signal value ($G_{922}$) are signal values in which the component of the observation target fluorescence is dominant. On the other hand, the signal value ($B_{922}$) is a signal value in which a component of unnecessary light is dominant. In this case, the signal value ($R_{922}$) and the signal value ($G_{922}$) correspond to the first pixel signal (signal value) according to the present disclosure. In addition, the signal value ($B_{922}$) corresponds to the second pixel signal (signal value) according to the present disclosure. In this case, "$R_{922}*G_{922}$" obtained by multiplying the signal value ($R_{922}$) and the signal value ($G_{922}$) is to be a value in which the component of the observation target fluorescence is extremely dominant. By including the "$R_{922}*G_{922}$" in the matrix element in the input matrix $M_{922}$ (for example, Formulas (4) to (7)), it is possible to generate the corrected pixel signal that has efficiently suppressed the component of the unnecessary light.

In the fourth modification, the correction matrix $M_C$ is calculated as illustrated in the flowchart of FIG. 13.

First, the operator brings the distal end of the insertion unit 2 close to the white chart WC (FIG. 14), and causes the imaging unit 51 to capture the white chart WC in a state where the white chart WC is irradiated with the first light emitted from the light source device 3 (step S1). Here, the white chart WC is a white chart in which the light reflectance is approximately 1. That is, the first light (excitation light) reflected by the white chart WC and the unnecessary light generated in the observation optical path P0 are incident on the imaging unit 51. Among them, the first light (excitation light) is partially, substantially, or completely suppressed by the excitation light cut filter 511 in the imaging unit 51. That is, only unnecessary light is incident on the image sensor 513 via the color filter 514.

After step S1, the operator uses the image processing unit 92 to execute the WB processing (step S2) and the demosaic processing (step S3) on the captured image generated by the imaging unit 51, thereby acquiring the signal values ($R_{922}$, $G_{922}$, and $B_{922}$) for each pixel in the captured image. Subsequently, the operator averages the signal values ($R_{922}$, $G_{922}$, and $B_{922}$) for each pixel in the area Ar (FIG. 14) including the center in the white chart WC for each of the signal value ($R_{922}$), the signal value ($G_{922}$), and the signal value ($B_{922}$), thereby acquiring average signal values ($R_{922\_AVE}$, $G_{922\_AVE}$, and $B_{922\_AVE}$) (step S4).

After step S4, the operator selects the type of the input matrix $M_{922}$ (step S5).

Specifically, when calculating the correction matrix $M_C$ of 3*3, the operator selects the input matrix $M_{922}$ of Formula (1). When calculating the correction matrix $M_C$ of 3*8, the operator selects the input matrix $M_{922}$ of Formula (4). When calculating the correction matrix $M_C$ of 3*7, the operator selects the input matrix $M_{922}$ of Formula (5). When calculating the correction matrix $M_C$ of 3*6, the operator selects one of the three input matrices $M_{922}$ of Formula (6). When calculating the correction matrix $M_C$ of 3*5, the operator selects one of the three input matrices $M_{922}$ of Formula (7). When calculating the correction matrix $M_C$ of 3*4, the operator selects the input matrix $M_{922}$ of Formula (8).

After step S5, the operator calculates the input matrix $M_{922}$ selected in step S5 from the average signal values ($R_{922\_AVE}$, $G_{922\_AVE}$, and $B_{922\_AVE}$) acquired in step S4 (step S6).

After step S6, the operator inputs the input matrix $M_{922}$ calculated in step S6 to the following Formula (9) (step S7). That is, step S7 generates Formula (9) in which the correction matrix $M_C$ is a variable.

$$\begin{pmatrix} 0 \\ 0 \\ 0 \end{pmatrix} = M_C * M_{922} \qquad \cdots \ (9)$$

After step S7, the operator uses the least squares error method to calculate the correction matrix $M_C$ from Formula (9) generated in step S7 in which the correction matrix $M_C$ is a variable (step S8). The method of calculating the correction matrix $M_C$ in step S8 is not limited to the least squares error method, and other methods may be adopted.

As described above, only unnecessary light is incident to the image sensor 513 via the color filter 514. Accordingly, the matrix elements in the input matrix $M_{922}$ include only the component of the unnecessary light. By setting the left side to "0" in Formula (9), step S8 calculates the correction matrix $M_C$ for removing only the component of the unnecessary light.

The fourth modification described above achieves the following effect in addition to effects similar to those of the embodiment described above.

In the fourth modification, the correction matrices $M_C$ of 3*8, 3*7, 3*6, 3*5, and 3*4 can be used in addition to the correction matrix $M_C$ of 3*3. That is, the number of correction parameters is increased, making it possible to generate a corrected pixel signal that has effectively suppressed the component of unnecessary light. In addition, by including the value multiplied by each signal value of at least two first pixel signals in the matrix element in the input matrix $M_{922}$, it is possible to generate a corrected pixel signal that has efficiently suppressed the component of the unnecessary light.

In the above-described embodiment and the present fourth modification, the second pixel signals according to the present disclosure have two signal values ($G_{922}$) and ($B_{922}$), and thus, the correction matrix $M_C$ of 3*n (n is 3 or more) is adopted. However, when the number of second pixel signals is only one, it is also allowable to adopt the correction matrix $M_C$ of 2*m (m is 2 or more).

(Fifth modification)

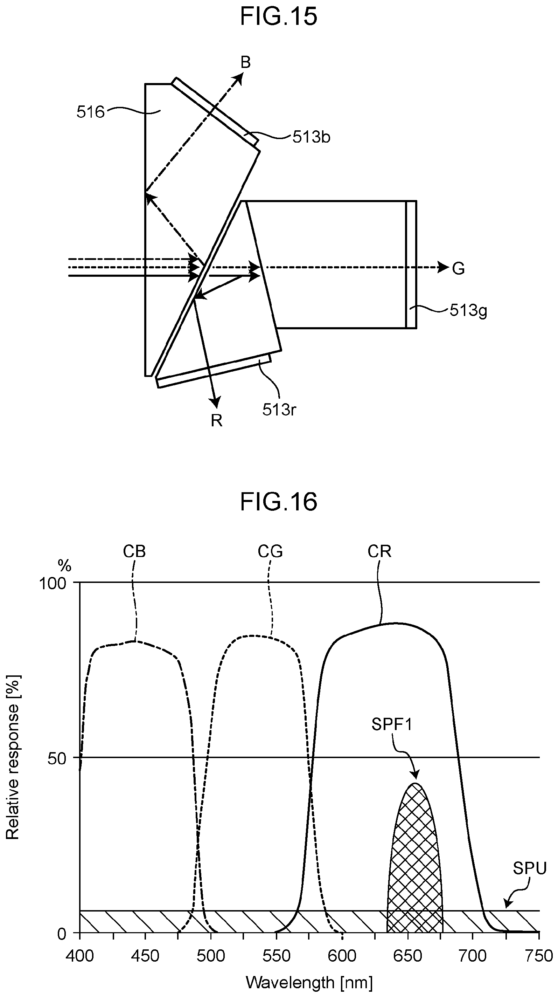

FIGS. 15 and 16 are diagrams each illustrating a fifth modification of the embodiment. Specifically, FIG. 15 is a diagram illustrating a configuration of a prism 516 and image sensors 513r, 513g, and 513b according to the fifth modification. In FIG. 15, the light in the red wavelength band spectrally dispersed by the prism 516 is denoted as "R", the light in the green wavelength band spectrally dispersed by the prism 516 is denoted as "G", and the light in the blue wavelength band spectrally dispersed by the prism 516 is denoted as "B". FIG. 16 is a diagram illustrating spectral characteristics of the prism 516. The spectral characteristic of the prism such as the prism 516 is also referred to as a wavelength spectral characteristic. In FIG. 16, the horizontal axis represents Wavelength[nm], and the vertical axis represents Relative response [%]. In addition, in FIG. 16, as the spectral characteristics of the prism 516, the spectrum of the light in the red wavelength band spectrally dispersed by the prism 516 is indicated by a curve CR, the spectrum of the light in the green wavelength band spectrally dispersed by the prism 516 is indicated by a curve CG, and the spectrum of the light in the blue wavelength band spectrally dispersed by the prism 516 is indicated by a curve CB. Note that, also in the fifth modification, as indicated by a spectrum SPF1 in FIG. 16, the spectral characteristic of the observation target fluorescence is the same spectral characteristic as that of the above-described embodiment, but the spectral characteristic may be other spectral characteristics. Similarly, the wavelength range SPU in which unnecessary light can be generated may be another wavelength range different from the range in the above-described embodiment.

In the above-described embodiment, the prism 516 and the image sensors 513r, 513g, and 513b according to the fifth modification illustrated in FIG. 15 may be adopted instead of the image sensor 513 and the color filter 514.

The prism 516 corresponds to a spectral dispersion member according to the present disclosure. The prism 516 spectrally disperses the second light (subject image) focused by the lens unit 512 after passing through the excitation light cut filter 511 into beams of light R, G, and B in the respective wavelength bands of red, green, and blue.

The image sensor 513r has a configuration similar to the configuration of the image sensor 513, and captures the light R in the red wavelength band spectrally dispersed by the prism 516.

The image sensor 513g has a configuration similar to the configuration of the image sensor 513, and captures the light G in the green wavelength band spectrally dispersed by the prism 516.

The image sensor 513b has a configuration similar to the configuration of the image sensor 513, and captures the light B in the blue wavelength band spectrally dispersed by the prism 516.

From the spectral characteristic (curve CR) of the prism 516 and the spectral characteristic of the observation target fluorescence and the unnecessary light, the signal value (r-value) of the pixel signal output from the image sensor 513r includes the component of the observation target fluorescence and the component of the unnecessary light. In addition, from the spectral characteristic (curve CG) of the prism 516 and the spectral characteristic of the observation target fluorescence and the unnecessary light, the signal value (g-value) of the pixel signal output from the image sensor 513g mainly includes the component of the unnecessary light. Furthermore, from the spectral characteristic (curve CB) of the prism 516 and the spectral characteristic of the observation target fluorescence and the unnecessary light, the signal value (b-value) of the pixel signal output from the image sensor 513b mainly includes the component of the unnecessary light. That is, the r-value includes a larger quantity of component of the observation target fluorescence in comparison with the g-value and the b-value. From the above, the pixel signal output from the image sensor 513r corresponds to the first pixel signal according to the present disclosure. In addition, the pixel signals output from the image sensors 513g and 513b correspond to second pixel signals according to the present disclosure.

The image processing unit 92 according to the fifth modification need not include the demosaic processing unit 922 described in the above-described embodiment. The correction matrix processing unit 923 according to the fifth modification performs the correction matrix processing using the signal value (r-value, g-value, b-value) obtained by performing the WB processing on the signal value ($R_{922}$, $G_{922}$, and $B_{922}$) of each pixel signal of the corresponding pixels from the image sensors 513r, 513g, and 513b, similarly to the above-described embodiment, thereby generating a corrected pixel signal. The correction matrix $M_C$ used in the correction matrix processing is set based on the spectral characteristic of the observation target fluorescence, the spectral characteristic of the unnecessary light, and the spectral characteristic of the prism 516.

Even when adopting the configuration of the fifth modification described above, it is possible to obtain the effects similar to those of the above-described embodiment.

In addition, the configuration of the fourth modification described above may be adopted in the fifth modification.

(Sixth modification)

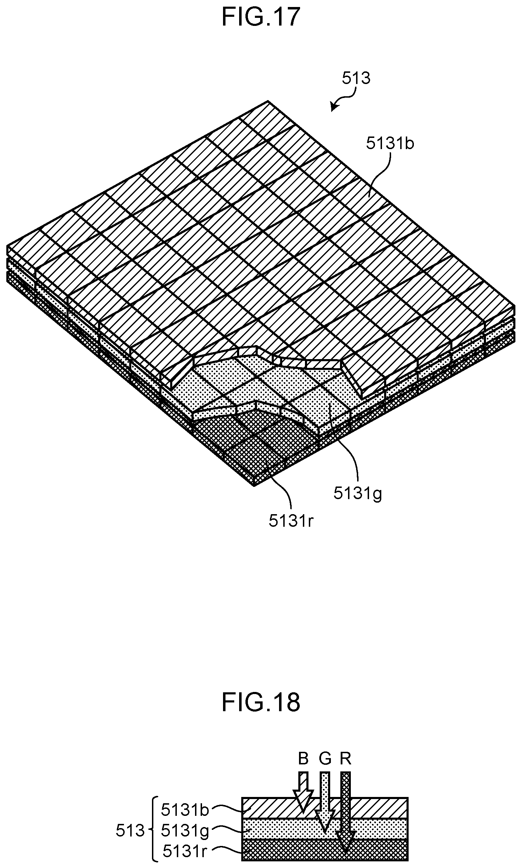

FIGS. 17 and 18 are diagrams each illustrating the sixth modification of the embodiment. Specifically, FIG. 17 is a perspective view illustrating an image sensor 513 according to the sixth modification. FIG. 18 is a side view illustrating the image sensor 513 according to the sixth modification. In FIG. 18, the light in the red wavelength band is denoted as "R", the light in the green wavelength band is denoted as "G", and the light in the blue wavelength band is denoted as "B".

In the above-described embodiment, the image sensor 513 according to the sixth modification illustrated in FIGS. 17 and 18 may be adopted instead of the image sensor 513 and the color filter 514.

The image sensor 513 according to the sixth modification is a Foveon type image sensor (for example, an organic CMOS). Specifically, as illustrated in FIGS. 17 and 18, the image sensor 513 has a configuration in which first to third light receiving layers 5131b, 5131g, and 5131r are stacked.

The first light receiving layer 5131b receives only light in a blue wavelength band and converts the light into an electrical signal. The first light receiving layer 5131b is provided in the uppermost layer, receives the light B in the blue wavelength band contained in the second light (subject image) focused by the lens unit 512 after passing through the excitation light cut filter 511, and converts the light B into an electrical signal.

The second light receiving layer 5131g receives only light in the green wavelength band and converts the light into an electrical signal. The second light receiving layer 5131g is provided below the first light receiving layer 5131b, receives the light G in the green wavelength band contained in the light transmitted through the first light receiving layer 5131b, and converts the light G into an electrical signal.

The third light receiving layer 5131r is provided in the lowermost layer, receives the light R in the red wavelength band transmitted through the first and second light receiving layers 5131b and 5131g, and converts the light R into an electrical signal.

For example, in a case where the spectral characteristic of the observation target fluorescence is the spectral characteristic of the wavelength band included in the red wavelength band of the light R, the electrical signal (pixel signal (r-value)) output from the third light receiving layer 5131r mainly includes the component of the observation target fluorescence, and thus corresponds to the first pixel signal according to the present disclosure. On the other hand, the electrical signals (pixel signals (g-value and b-value)) output from the first and second light receiving layers 5131b and 5131g mainly include a component of unnecessary light, and thus correspond to the second pixel signal according to the present disclosure.

The image processing unit 92 according to the sixth modification need not include the demosaic processing unit 922 described in the above-described embodiment. The correction matrix processing unit 923 according to the sixth modification performs the correction matrix processing using the signal value (r-value, g-value, b-value) obtained by performing the WB processing on the signal value ($R_{922}$, $G_{922}$, and $B_{922}$) of each pixel signal of the corresponding pixels from the first to third light receiving layers 5131b, 5131g, and 5131r, similarly to the above-described embodiment, thereby generating a corrected pixel signal. The correction matrix $M_C$ used in the correction matrix processing is set based on the spectral characteristic of the observation target fluorescence, the spectral characteristic of the unnecessary light, and the spectral characteristic of the image sensor 513 according to the sixth modification. The spectral characteristic of the Foveon type image sensor such as the image sensor 513 according to the sixth modification is also referred to as a light-receiving spectral characteristic.

Even when adopting the configuration of the sixth modification described above, it is possible to obtain the effects similar to those of the above-described embodiment.

In addition, the configuration of the fourth modification described above may be adopted in the sixth modification.

(Seventh modification)

The medical observation system according to the seventh modification is a medical observation system using a device referred to as a video scope (flexible endoscope) including an imaging unit on the distal end side of the insertion unit. Hereinafter, for convenience of description, the medical observation system 1 according to the seventh modification will be denoted as a medical observation system 1A.

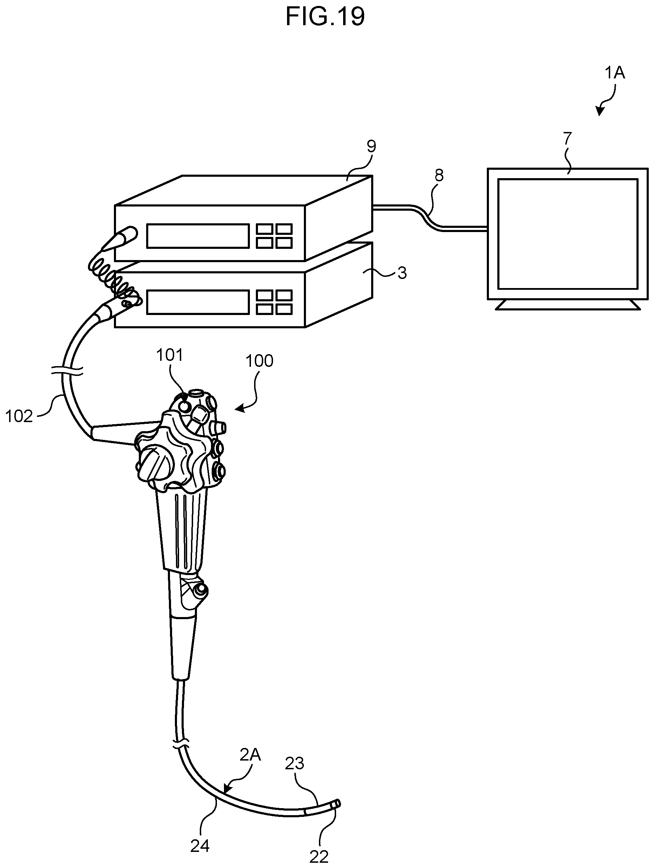

FIG. 19 is a diagram illustrating the seventh modification of the embodiment.

As illustrated in FIG. 19, the medical observation system 1A includes: an endoscope 100 that captures an in-vivo image of an observed region by inserting an insertion unit 2A into a living body and outputs the captured image; a light source device 3 that emits first light from the distal end of the endoscope 100; a control device 9 that processes the captured image output from the endoscope 100; and a display device 7 connected to the control device 9 via a second transmission cable 8 and configured to display an image based on a video signal processed by the control device 9.

As illustrated in FIG. 19, the endoscope 100 includes the insertion unit 2A that is a flexible and elongated portion, an operating unit 101 that is connected on a proximal end of the insertion unit 2A and receives input of various operation signals, and a universal cord 102 that extends from the operating unit 101 in a direction different from the extending direction of the insertion unit 2A and incorporates various cables for connecting with the light source device 3 and the control device 9.

As illustrated in FIG. 19, the insertion unit 2A includes: a distal end portion 22; a bendable portion 23 that is a bendable portion connected to the proximal end side of the distal end portion 22 and is formed with a plurality of bending pieces; and a flexible tube portion 24 that is a flexible and elongated portion connected to the proximal end side of the bendable portion 23.

Although the specific illustration is omitted, the distal end portion 22 incorporates a configuration substantially similar to that of the camera head 5 described above in the embodiment. The captured image captured by the distal end portion 22 (imaging unit) is output to the control device 9 via the operating unit 101 and the universal cord 102.

Even when adopting the configuration of the seventh modification described above, it is possible to obtain the effects similar to those of the above-described embodiment.

(Eighth modification)

A medical observation system according to an eighth modification is a medical observation system using a surgical microscope that enlarges and captures a predetermined visual field area inside the subject (inside the living body) or a surface of the subject (surface of the living body). Hereinafter, for convenience of description, the medical observation system 1 according to the seventh modification will be denoted as a medical observation system 1B.

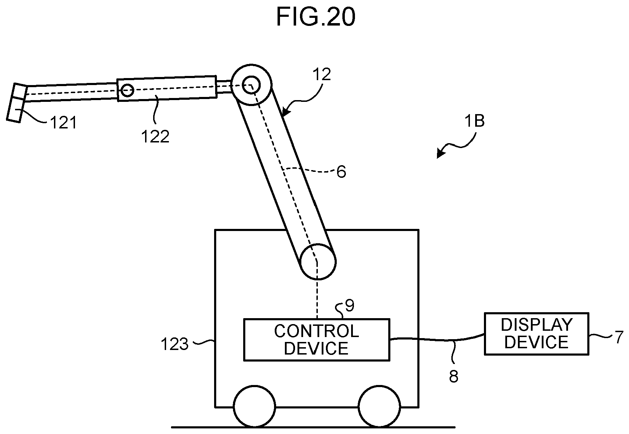

FIG. 20 is a diagram illustrating the eighth modification of the embodiment.

As illustrated in FIG. 20, the medical observation system 1B includes: a surgical microscope 12 that captures an image for observing the subject and outputs a captured image; a control device 9 that processes the captured image output from the surgical microscope 12; and a display device 7 that is connected to the control device 9 via a second transmission cable 8 and displays an image based on a video signal processed by the control device 9.

As illustrated in FIG. 20, the surgical microscope 12 includes: a microscope unit 121 that captures an enlarged image of the minute sites of the subject and outputs the captured image; a support 122 connected to the proximal end portion of the microscope unit 121 and that includes an arm that pivotally supports the microscope unit 121; and a base 123 that pivotally holds the proximal end portion of the support 122 and is movable on the floor surface.

As illustrated in FIG. 20, the control device 9 is installed in the base 123. In addition, although a specific illustration is omitted, a light source device 3 for generating illumination light to irradiate the subject from the surgical microscope 12 is also installed on the base 123.

The base 123 may also be fixed to the ceiling, wall surface, or the like to support the support 122, instead of being movably provided on the floor surface.

Although the specific illustration is omitted, the microscope unit 121 incorporates a configuration substantially similar to that of the camera head 5 described above in the embodiment. The captured image that has been captured by the imaging on the microscope unit 121 (imaging unit) is output to the control device 9 via a first transmission cable 6 wired along the support 122.

Even when adopting the configuration of the eighth modification described above, it is possible to obtain the effects similar to those of the above-described embodiment.

(Ninth modification)

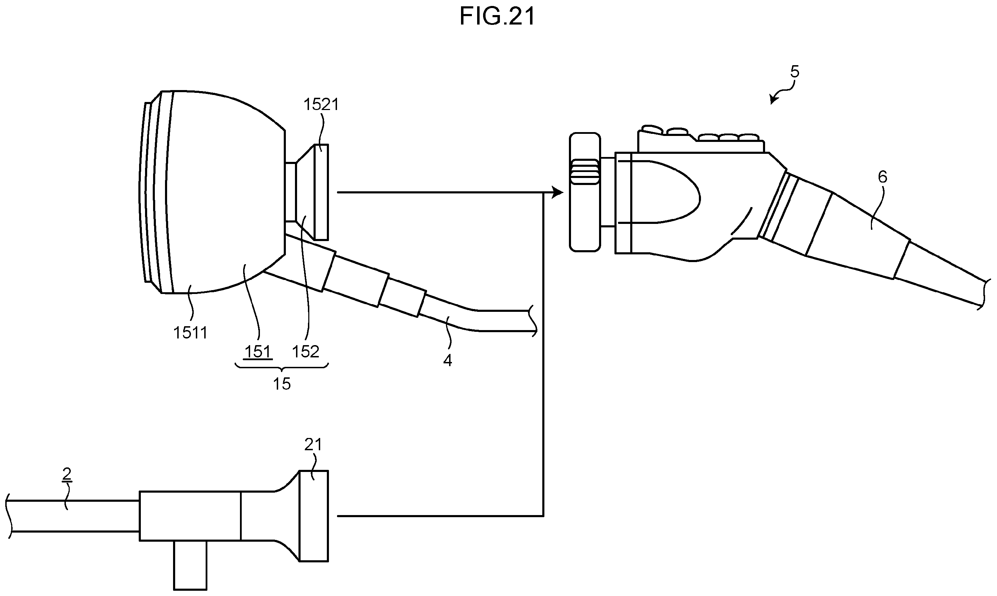

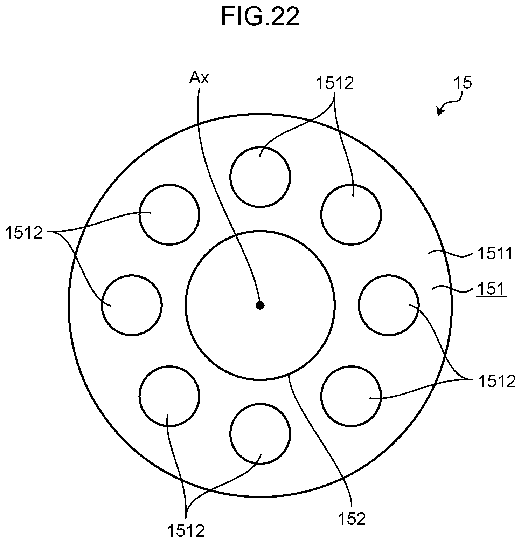

FIGS. 21 and 22 are diagrams each illustrating a ninth modification of the embodiment. Specifically, FIG. 21 is a side view of a ring light 15. The ring ride 15 corresponds to an open field observation device for observing a living tissue. The open field observation device is a device used in laparotomy. FIG. 22 is a diagram of the ring light 15 as viewed from the front side (left side in FIG. 21).

In the ninth modification, in addition to the insertion unit 2 in the embodiment described above, the ring light 15 illustrated in FIGS. 21 and 22 is detachably connected to the camera head 5. That is, as illustrated in FIG. 21, the insertion unit 2 may be connected to the camera head 5 or the ring light 15 may be connected to the camera head 5 depending on the use state of the user.

Instead of being inserted into the observation target OB like the insertion unit 2, the ring light 15 supplies the first light to the surgical site and captures the second light (subject image) which is return light of the first light from the surgical site. As illustrated in FIGS. 21 and 22, the ring light 15 includes an illumination unit 151 and a subject image capturing unit 152 that captures a subject image.

As illustrated in FIGS. 21 and 22, the illumination unit 151 includes a housing 1511 and a plurality of illumination lenses 1512.

The housing 1511 has an annular shape centered on an optical axis Ax. The light guide 4 has its other end detachably connected to the housing 1511.

As illustrated in FIG. 22, the plurality of illumination lenses 1512 is arranged at predetermined intervals in a circumferential direction centered on the optical axis Ax on the end surface on the front side of the housing 1511. Subsequently, the plurality of illumination lenses 1512 irradiate the surgical site with the first light supplied from the light source device 3 and introduced into the housing 1511 via the light guide 4.

The subject image capturing unit 152 extends along the optical axis Ax. The subject image capturing unit 152 internally includes an optical system formed with one or more lenses and configured to condense the second light (subject image) emitted from the plurality of illumination lenses 1512 and passing through the surgical site. Furthermore, there is provided a connection unit 1521 at an end portion on the proximal end side (right side in FIG. 21) of the subject image capturing unit 152. The connection unit 1521 is designed (shaped) to be compatible with the eyepiece 21 in the insertion unit 2, and is detachably connected to the camera head 5.

Even when adopting the configuration of the ninth modification described above, it is possible to obtain the effects similar to those of the above-described embodiment.

(Tenth modification)

In the above-described embodiment, the observation target fluorescence is visible light, but the observation target fluorescence is not limited thereto, and may be invisible light.

At this time, as the image sensor according to the tenth modification, as in the image sensor 513 of the above-described embodiment, the number of the image sensors may be one as long as the image sensor has sensitivity capable of imaging not only visible light but also invisible light, and can also image observation target fluorescence of the invisible light.

In addition, it is also allowable to adopt, as the image sensor according to the tenth modification, an image sensor that captures only invisible light, such as an infrared image sensor. At this time, it is also allowable to use a configuration including one image sensor that captures only the invisible light as long as the image sensor has sensitivity to each of a plurality of wavelength bands and each can output a first pixel signal mainly including the component of the observation target fluorescence of the invisible light and a second pixel signal mainly including the component of the unnecessary light of the invisible light. In addition, it is also allowable to have a configuration in which two image sensors of an image sensor that captures invisible light and an image sensor that captures visible light are provided, and in which observation target fluorescence of invisible light is captured by the image sensor that captures invisible light, and unnecessary light of visible light is captured by the image sensor that captures visible light.

Even when adopting the configuration of the tenth modification described above, it is possible to obtain the effects similar to those of the above-described embodiment.

The following configurations also belong to the technical scope of the present disclosure.

(1) A medical image processing device including an image processing unit configured to process a pixel signal acquired from a pixel of an image sensor, wherein the pixel signal includes: a first pixel signal including a component of unnecessary light and a component of observation target fluorescence, the unnecessary light including autofluorescence which is generated from a member forming an observation optical path by light emitted from a light source device when the light propagates through the observation optical path, the component of observation target fluorescence having been emitted from a substance contained in an observation target excited by the light; and a second pixel signal including at least the component of the unnecessary light, and the image processing unit includes a signal correction unit configured to generate a corrected pixel signal based on a correction coefficient, the first pixel signal and the second pixel signal, the correction coefficient having been set based on a spectral characteristic of the observation target fluorescence.

(2) The medical image processing device according to (1), wherein the component of the observation target fluorescence is included in the first pixel signal by a larger quantity than in the second pixel signal.

(3) The medical image processing device according to (1) or (2), wherein the signal correction unit is configured to generate the corrected pixel signal by multiplying a correction matrix being the correction coefficient by an input matrix, the input matrix including a signal value of the first pixel signal and a signal value of the second pixel signal as matrix elements.

(4) The medical image processing device according to (3), wherein the first pixel signal is provided as at least two first pixel signals, and the input matrix includes, as the matrix element, a value obtained by multiplying signal values of the at least two first pixel signals.

(5) The medical image processing device according to (3), wherein the matrix element to be multiplied by a signal value of the first pixel signal in the correction matrix is set to 0 or a positive value, and the matrix element to be multiplied by a signal value of the second pixel signal in the correction matrix is set to 0 or a negative value.

(6) The medical image processing device according to (5), wherein, in each row of the correction matrix, each number obtained by adding matrix elements is 0.

(7) The medical image processing device according to (5), wherein, in each row of the correction matrix, each number obtained by adding matrix elements is larger than 0.

(8) The medical image processing device according to (5), wherein, in each row of the correction matrix, each number obtained by adding matrix elements is smaller than 0.

(9) The medical image processing device according to any one of (1) to (8), wherein the image sensor is configured to capture each beam of light obtained by spectrally dispersing light from the observation target by a spectral dispersion member, and the correction coefficient has been set based on the spectral characteristic of the observation target fluorescence and a spectral characteristic of the spectral dispersion member.

(10) The medical image processing device according to any one of (1) to (9), wherein the correction coefficient has been set based on the spectral characteristic of the observation target fluorescence and a spectral characteristic of the unnecessary light.

(11) The medical image processing device according to (9), wherein the image processing unit further includes a spectral sensitivity correction unit configured to perform spectral sensitivity correction processing on the first pixel signal and the second pixel signal based on the spectral characteristic of the spectral dispersion member, and the signal correction unit is configured to generate the corrected pixel signal by using the first pixel signal and the second pixel signal after the spectral sensitivity correction processing has been executed by the spectral sensitivity correction unit.

(12) The medical image processing device according to any one of (1) to (11), wherein the first pixel signal and the second pixel signal are acquired from the same image sensor.

(13) The medical image processing device according to any one of (1) to (11), wherein the image sensor is provided in plurality, and the first pixel signal and the second pixel signal are acquired from different image sensors.

(14) The medical image processing device according to any one of (1) to (13), wherein the observation target fluorescence is generated from an agent or a fluorescent dye applied to the observation target, or from a fluorescent substance derived from the observation target.

(15) The medical image processing device according to any one of (1) to (14), wherein the member forming the observation optical path and being configured to generate the autofluorescence is an optical fiber, a lens, glass, a bonding material or a filter.

(16) The medical image processing device according to any one of (1) to (15), wherein the corrected pixel signal is a signal that has reduced a signal value based on the unnecessary light.

(17) A medical observation system including: a light source device including a first light source configured to emit first light including narrowband light that excites a substance contained in an observation target; an imaging device including an image sensor configured to capture second light that is return light of the first light from the observation target; and a medical image processing device including an image processing unit configured to process a pixel signal corresponding to a pixel of the image sensor, wherein the pixel signal includes: a first pixel signal including a component of unnecessary light and a component of observation target fluorescence, the unnecessary light including autofluorescence which is generated from a member forming an observation optical path by the first light emitted from the light source device when the first light propagates through the observation optical path, the component of observation target fluorescence having been emitted from a substance contained in the observation target excited by the first light; and a second pixel signal including at least the component of the unnecessary light, and the image processing unit includes a signal correction unit configured to generate a corrected pixel signal based on a correction coefficient, the first pixel signal, and the second pixel signal, the correction coefficient having been set based on a spectral characteristic of the observation target fluorescence.

(18) The medical observation system according to (17),
wherein the first light source is provided as one or more light sources.

(19) The medical observation system according to (17) or (18), wherein the first light source is provided as an LED or a semiconductor laser.

(20) The medical observation system according to any one of (17) to (19), wherein the first light is provided as broadband light or narrowband light.

Reference Signs List

| | |
|---|---|
| 1, 1A, 1B | MEDICAL OBSERVATION SYSTEM |
| 2, 2A | INSERTION UNIT |
| 3 | LIGHT SOURCE DEVICE |
| 4 | LIGHT GUIDE |
| 5 | CAMERA HEAD |
| 6 | FIRST TRANSMISSION CABLE |
| 7 | DISPLAY DEVICE |
| 8 | SECOND TRANSMISSION CABLE |
| 9 | CONTROL DEVICE |
| 10 | THIRD TRANSMISSION CABLE |
| 12 | SURGICAL MICROSCOPE |
| 15 | RING LIGHT |
| 21 | EYEPIECE |
| 22 | DISTAL END PORTION |
| 23 | BENDABLE PORTION |
| 24 | FLEXIBLE TUBE PORTION |
| 31 | FIRST LIGHT SOURCE |
| 51 | IMAGING UNIT |
| 52 | COMMUNICATION UNIT |
| 91 | COMMUNICATION UNIT |
| 92 | IMAGE PROCESSING UNIT |
| 93 | CONTROL UNIT |
| 94 | INPUT UNIT |
| 95 | OUTPUT UNIT |
| 96 | STORAGE UNIT |
| 100 | ENDOSCOPE |

| | |
|---|---|
| 101 | OPERATING UNIT |
| 102 | UNIVERSAL CORD |
| 121 | MICROSCOPE UNIT |
| 122 | SUPPORT |
| 123 | BASE |
| 151 | ILLUMINATION UNIT |
| 152 | SUBJECT IMAGE CAPTURING UNIT |
| 511 | EXCITATION LIGHT CUT FILTER |
| 512 | LENS UNIT |
| 513, 513b, 513g, 513r | IMAGE SENSOR |
| 514 | COLOR FILTER |
| 514b | B FILTER GROUP |
| 514g | G FILTER GROUP |
| 514r | R FILTER GROUP |
| 515 | SIGNAL PROCESSING UNIT |
| 516 | PRISM |
| 921 | WB PROCESSING UNIT |
| 922 | DEMOSAIC PROCESSING UNIT |
| 923 | CORRECTION MATRIX PROCESSING UNIT |
| 924 | GAMMA PROCESSING UNIT |
| 925 | YC CONVERTER |
| 1511 | HOUSING |
| 1512 | ILLUMINATION LENS |
| 5131b | FIRST LIGHT RECEIVING LAYER |
| 5131g | SECOND LIGHT RECEIVING LAYER |
| 5131r | THIRD LIGHT RECEIVING LAYER |
| Ar | AREA |
| Ar1 | FIRST AREA |
| Ar2 | SECOND AREA |
| Ax | OPTICAL AXIS |
| CB, CG, CR | CURVE |
| CN1, CN2 | CONNECTOR |
| DG | AGENT |
| F1 | CAPTURED IMAGE |
| FR, FG, FB | FREQUENCY DISTRIBUTION |
| OB | OBSERVATION TARGET |
| P0 | OBSERVATION OPTICAL PATH |
| P1 | FIRST OPTICAL PATH |
| P2 | SECOND OPTICAL PATH |
| SPF1, SPF2, SPF3, SPF4 | SPECTRUM OF OBSERVATION TARGET FLUORESCENCE |
| SPU | WAVELENGTH RANGE IN WHICH UNNECESSARY LIGHT can be generated |
| TU | TEST TUBE |
| WC | WHITE CHART |

**Claims**

1. A medical image processing device comprising

   an image processing unit configured to process a pixel signal acquired from a pixel of an image sensor, wherein the pixel signal includes:

   a first pixel signal including a component of unnecessary light and a component of observation target fluorescence, the unnecessary light including autofluorescence which is generated from a member forming an observation optical path by light emitted from a light source device when the light propagates through the observation optical path, the component of observation target fluorescence having been emitted from a substance contained in an observation target excited by the light; and
   a second pixel signal including at least the component of the unnecessary light, and

the image processing unit includes a signal correction unit configured to generate a corrected pixel signal based on a correction coefficient, the first pixel signal and the second pixel signal, the correction coefficient having been set based on a spectral characteristic of the observation target fluorescence.

2.  The medical image processing device according to claim 1, wherein the component of the observation target fluorescence is included in the first pixel signal by a larger quantity than in the second pixel signal.

3.  The medical image processing device according to claim 1, wherein the signal correction unit is configured to generate the corrected pixel signal by multiplying a correction matrix being the correction coefficient by an input matrix, the input matrix including a signal value of the first pixel signal and a signal value of the second pixel signal as matrix elements.

4.  The medical image processing device according to claim 3, wherein

    the first pixel signal is provided as at least two first pixel signals, and
    the input matrix includes, as the matrix element, a value obtained by multiplying signal values of the at least two first pixel signals.

5.  The medical image processing device according to claim 3, wherein

    the matrix element to be multiplied by a signal value of the first pixel signal in the correction matrix is set to 0 or a positive value, and
    the matrix element to be multiplied by a signal value of the second pixel signal in the correction matrix is set to 0 or a negative value.

6.  The medical image processing device according to claim 5, wherein, in each row of the correction matrix, each number obtained by adding matrix elements is 0.

7.  The medical image processing device according to claim 5, wherein, in each row of the correction matrix, each number obtained by adding matrix elements is larger than 0.

8.  The medical image processing device according to claim 5, wherein, in each row of the correction matrix, each number obtained by adding matrix elements is smaller than 0.

9.  The medical image processing device according to claim 1, wherein

    the image sensor is configured to capture each beam of light obtained by spectrally dispersing light from the observation target by a spectral dispersion member, and
    the correction coefficient has been set based on the spectral characteristic of the observation target fluorescence and a spectral characteristic of the spectral dispersion member.

10. The medical image processing device according to claim 1, wherein the correction coefficient has been set based on the spectral characteristic of the observation target fluorescence and a spectral characteristic of the unnecessary light.

11. The medical image processing device according to claim 9, wherein

    the image processing unit further includes a spectral sensitivity correction unit configured to perform spectral sensitivity correction processing on the first pixel signal and the second pixel signal based on the spectral characteristic of the spectral dispersion member, and
    the signal correction unit is configured to generate the corrected pixel signal by using the first pixel signal and the second pixel signal after the spectral sensitivity correction processing has been executed by the spectral sensitivity correction unit.

12. The medical image processing device according to claim 1, wherein the first pixel signal and the second pixel signal are acquired from the same image sensor.

13. The medical image processing device according to claim 1, wherein

the image sensor is provided in plurality, and
the first pixel signal and the second pixel signal are acquired from different image sensors.

14. The medical image processing device according to claim 1, wherein the observation target fluorescence is generated from an agent or a fluorescent dye applied to the observation target, or from a fluorescent substance derived from the observation target.

15. The medical image processing device according to claim 1, wherein the member forming the observation optical path and being configured to generate the autofluorescence is an optical fiber, a lens, glass, a bonding material or a filter.

16. The medical image processing device according to claim 1, wherein the corrected pixel signal is a signal that has reduced a signal value based on the unnecessary light.

17. A medical observation system comprising:

a light source device including a first light source configured to emit first light including narrowband light that excites a substance contained in an observation target;
an imaging device including an image sensor configured to capture second light that is return light of the first light from the observation target; and
a medical image processing device including an image processing unit configured to process a pixel signal corresponding to a pixel of the image sensor,
wherein the pixel signal includes:

a first pixel signal including a component of unnecessary light and a component of observation target fluorescence, the unnecessary light including autofluorescence which is generated from a member forming an observation optical path by the first light emitted from the light source device when the first light propagates through the observation optical path, the component of observation target fluorescence having been emitted from a substance contained in the observation target excited by the first light; and
a second pixel signal including at least the component of the unnecessary light, and

the image processing unit includes a signal correction unit configured to generate a corrected pixel signal based on a correction coefficient, the first pixel signal, and the second pixel signal, the correction coefficient having been set based on a spectral characteristic of the observation target fluorescence.

18. The medical observation system according to claim 17, wherein the first light source is provided as one or more light sources.

19. The medical observation system according to claim 17, wherein the first light source is provided as an LED or a semiconductor laser.

20. The medical observation system according to claim 17, wherein the first light is provided as broadband light or narrowband light.

# FIG.1

FIG.2

# FIG.3

# FIG.4

## FIG.5

## FIG.6

# FIG.7

F1

TU

DR

Ar1

Ar2

## FIG.8

(a)

FR,FB    FG

NUMBER OF PIXELS

r-VALUE, g-VALUE, AND b-VALUE

(b)

FR,FG,FB

NUMBER OF PIXELS

r-VALUE, g-VALUE, AND b-VALUE

## FIG.9

## FIG.10

# FIG.11

# FIG.12

# FIG.13

START

IMAGING  $\varsigma$ S1

WB PROCESSING  $\varsigma$ S2

DEMOSAIC PROCESSING  $\varsigma$ S3

ACQUIRE AVERAGE SIGNAL VALUE  $\varsigma$ S4

SELECT TYPE OF INPUT MATRIX $M_{922}$  $\varsigma$ S5

CALCULATE INPUT MATRIX $M_{922}$  $\varsigma$ S6

CALCULATE CORRECTION MATRIX $M_C$ BY LEAST SQUARES ERROR METHOD  $\varsigma$ S7

END

# FIG.14

WC

Ar

# FIG.15

# FIG.16

EP 4 772 103 A1

## FIG.17

## FIG.18

36

# FIG.19

# FIG.20

FIG.21

EP 4 772 103 A1

## FIG.22

# EP 4 772 103 A1

| INTERNATIONAL SEARCH REPORT | International application No. |
| --- | --- |
| | **PCT/JP2024/023815** |

**A.    CLASSIFICATION OF SUBJECT MATTER**

*A61B 1/00*(2006.01)i; *A61B 1/045*(2006.01)i
FI:    A61B1/00 511; A61B1/045 611

According to International Patent Classification (IPC) or to both national classification and IPC

**B.    FIELDS SEARCHED**

Minimum documentation searched (classification system followed by classification symbols)

A61B1/00-1/32; G01N21/64; G02B21/00-21/36

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2008-229026 A (OLYMPUS CORPORATION) 02 October 2008 (2008-10-02) paragraphs [0001]-[0036], fig. 1-3 | 1, 3, 5-20 |
| A | | 2, 4 |
| A | JP 2020-78543 A (CANON U.S.A., INC.) 28 May 2020 (2020-05-28) paragraphs [0054]-[0057], fig. 6-7 | 1-20 |
| A | WO 2014/091792 A1 (KONICA MINOLTA, INC.) 19 June 2014 (2014-06-19) paragraph [0065] | 1-20 |
| A | JP 2006-10862 A (OLYMPUS CORPORATION) 12 January 2006 (2006-01-12) paragraph [0005] | 1-20 |
| A | JP 2008-203138 A (CANON KABUSHIKI KAISHA) 04 September 2008 (2008-09-04) entire text, all drawings | 1-20 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **02 September 2024** | **17 September 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 772 103 A1

| INTERNATIONAL SEARCH REPORT | | International application No. |
| :---: | :---: | :---: |
| Information on patent family members | | **PCT/JP2024/023815** |

| Patent document cited in search report | | | Publication date (day/month/year) | Patent family member(s) | | | Publication date (day/month/year) |
| :---: | :---: | :---: | :---: | :---: | :---: | :---: | :---: |
| JP | 2008-229026 | A | 02 October 2008 | US 2010/0036262 A1 paragraphs [0001]-[0069], fig. 1-3 WO 2008/114735 A1 | | | |
| JP | 2020-78543 | A | 28 May 2020 | US 2020/0085285 A1 paragraphs [0064]-[0067], fig. 6A-7B | | | |
| WO | 2014/091792 | A1 | 19 June 2014 | JP 5505578 B1 | | | |
| JP | 2006-10862 | A | 12 January 2006 | US 2007/0167841 A1 paragraph [0009] WO 2006/001314 A1 EP 1760512 A1 CN 1973234 A | | | |
| JP | 2008-203138 | A | 04 September 2008 | US 2008/0198368 A1 entire text, all drawings | | | |

Form PCT/ISA/210 (patent family annex) (July 2022)

42

**EP 4 772 103 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021132695 A